(19) Europäisches Patentamt
European Patent Office
Office européen des brevets

(11) EP 1 226 210 B1

(12) **FASCICULE DE BREVET EUROPEEN**

(45) Date de publication et mention de la délivrance du brevet:
**17.12.2003 Bulletin 2003/51**

(21) Numéro de dépôt: **00967974.7**

(22) Date de dépôt: **06.10.2000**

(51) Int Cl.[7]: **C08K 5/55**, C08L 83/04

(86) Numéro de dépôt international:
**PCT/FR00/02789**

(87) Numéro de publication internationale:
**WO 0103/0903 (03.05.2001 Gazette 2001/18)**

(54) **UTILISATION D'AMORCEURS DE POLYMERISATION ET/OU RETICULATION DE POLYORGANOSILOXANES A GROUPEMENTS FONCTIONNELS RETICULABLES, COMPOSITIONS CORRESPONDANTES**

VERWENDUNG VON BESCHLEUNIGERN FÜR DIE POLYMERISATION UND/ODER VERNETZUNG VON POLYORGANOSILOXANEN MIT VERNETZBAREN FUNKTIONELLEN GRUPPEN, ENTSPRECHENDE ZUSAMMENSETZUNGEN

USE OF INITIATORS FOR POLYMERISING AND/OR CROSS-LINKING POLYORGANOSILOXANES WITH CROSS-LINKABLE FUNCTIONAL GROUPS, CORRESPONDING COMPOSITIONS

(84) Etats contractants désignés:
**AT BE CH CY DE DK ES FI FR GB GR IE IT LI LU MC NL PT SE**

(30) Priorité: **29.10.1999 FR 9913620**

(43) Date de publication de la demande:
**31.07.2002 Bulletin 2002/31**

(73) Titulaire: **RHODIA CHIMIE**
**92512 Boulogne Billancourt Cedex (FR)**

(72) Inventeurs:
- **FRANCES, Jean-Marc**
  **F-69330 Meyzieu (FR)**
- **DEFORTH, Thomas**
  **F-69007 Lyon (FR)**

(74) Mandataire: **Jacobson, Claude**
**Cabinet Lavoix**
**2, Place d'Estienne d'Orves**
**75441 Paris Cedex 09 (FR)**

(56) Documents cités:
**US-A- 4 772 325** **US-A- 5 973 020**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne le domaine de l'amorçage des réactions de polymérisation(s) et/ou de réticulation(s) de monomères, oligomères et/ou polymères de nature polyorganosiloxane et comprenant des radicaux fonctionnels réactifs aptes à former des pontages intra- et inter-caténaires, de manière à obtenir une matrice correspondante.

Ce type de matrice est particulièrement intéressant pour élaborer des compositions multiples tels matériaux dentaires, adhésifs, produits d'étanchéification, de jointage et les apprêts d'adhésion.

Les applications plus particulièrement visées selon l'invention sont l'utilisation de compositions pour la préparation de compositions dentaires comme les prothèses dentaires ou matériaux de restauration dentaire et de compositions de type revêtement anti-adhésif utiles notamment pour réaliser des revêtements sur objets tels que des articles ou des supports solides, notamment support papier, tissu, film polymère de type polyester ou polyoléfine, support aluminium, et/ou support métallique tel fer blanc.

Plus précisément, la présente invention a pour objet l'utilisation d'amorceurs comprenant au moins un dérivé du bore permettant l'initiation et le déroulement de réactions de formation de résines ou polymères, à partir de substrats dérivant de monomères, oligomères et/ou de polymères de nature polyorganosiloxane à groupements organofonctionnels réactifs.

Les réactions plus particulièrement concernées sont celles dans lesquelles des agents agissent comme promoteurs directs des liaisons inter- et/ou intra-caténaires. Dans le cas présent, ces réactions sont initiées sous activation thermique.

Dans la présente description, les résines et polymères obtenus sont préparés à partir de monomères, oligomères et/ou polymères de nature polyorganosiloxane, et comprennent dans leur structure des groupements organofonctionnels, par exemple du type époxyde, oxétane, dioxolane et/ou alcénylléther, qui réagissent après activation des amorceurs selon l'invention décrits ci-après. On peut également en plus utiliser des monomères, oligomères et/ou polymères organiques qui peuvent être ajoutés dans le milieu de polymérisation contenant les espèces précédentes.

La présente invention a également pour objet des compositions comprenant les matériaux de base comme des monomères, oligomères et/ou polymères de nature polyorganosiloxane réticulables, les amorceurs décrits ci-après, et éventuellement un ou plusieurs additifs choisis parmi ceux généralement connus dans les applications auxquelles sont destinées ces compositions.

Les amorceurs généralement utilisés pour initier la formation de résines ou de polymères à partir de la polymérisation et/ou réticulation des substrats organosiloxanes évoqués précédemment peuvent être divisés en trois catégories, en fonction de leur mode d'activation. Ce mode d'activation peut être en effet soit thermique, photochimique ou sous faisceau d'électrons.

Classiquement, lors de la réticulation photochimique tel rayonnement UV, l'amorceur utilisé, généralement un photoamorceur cationique, libère un acide fort sous irradiation. C'est cet acide fort qui catalyse la réaction de polymérisation cationique des groupements fonctionnels. Des amorceurs de ce type sont notamment décrits dans EP-0 562 897. Les sels amorceurs de ce brevet représentent un progrès technique notable par rapport aux amorceurs antérieurement connus de type sels d'onium ou de complexes organométalliques, et en particulier par rapport à ceux dont l'anion du sel amorceur est $SbF_6$- qui est l'un des seuls qui soient corrects sur le plan des performances, mais qui pose de graves problèmes d'utilisation en raison de la présence de métaux lourds.

Pour ce qui est de la réticulation par voie thermique, elle nécessite pour sa part des températures très élevées, généralement supérieures à 150°C pour déclencher la réticulation. Ce second type d'amorceur est notamment utilisé pour réticuler des silicones tels que décrits ci-après (S1 - S15) en couche mince et pour des époxydes ou oxétanes.

L'un des objectifs essentiels de la présente invention est précisément de proposer l'utilisation d'amorceurs activables thermiquement, permettant de déclencher à une température inférieure à 150°C et de préférence inférieure à 100°C voire de l'ordre de la température ambiante, la réticulation de monomères, oligomères et/ou polymères de nature poly-organosiloxane à groupements fonctionnels.

Plus particulièrement, la présente invention a pour premier objet l'utilisation d'amorceur de polymérisation et/ou de réticulation activable thermiquement, de monomères, oligomères et/ou de polymères à groupements organofonctionnels comprenant un dérivé du bore de formule I :

$$(A)_xB(R')_y \qquad (I)$$

Dans laquelle

- les symboles R' sont identiques ou différents et représentent

- un radical alkyle ou alcényle en $C_1$-$C_{12}$, de préférence en $C_1$-$C_8$, linéaire ou ramifié, éventuellement substitué par au moins un élément électroattracteur, notamment un atome d'halogène (fluor tout particulièrement), ou un groupement électroattracteur comme par exemple les groupements $CF_3$, $NO_2$, CN,

- un radical alkoxy en $C_1$-$C_{12}$, de préférence en $C_1$-$C_8$, linéaire ou ramifié, éventuellement substitué par au moins un élément électroattracteur, notamment un atome d'halogène (le fluor tout particulièrement), ou un groupement électroattracteur comme par exemple les groupements $CF_3$, $NO_2$, CN,

- un radical phényle substitué par au moins un élément électroattracteur, notamment un atome d'halogène (fluor tout particulièrement), ou un groupement électroattracteur, notamment un groupement $CF_3$, $NO_2$, CN,

- un radical aryle contenant au moins deux noyaux aromatiques tel que biphényle, naphtyle, éventuellement substitué par au moins un élément éectroattracteur, notamment atome d'halogène (fluor tout particulièrement), ou un groupement électroattracteur notamment un groupement $CF_3$, $NO_2$, CN,

- un radical $-C_2H_4-SI(Q)_3$ avec les symboles Q identiques ou différents représentant un groupe alkyle ou alkoxy en $C_1$ à $C_{10}$ ou un oligomère siloxane inférieur à 10 atomes de silicium, le cas échéant substitué par un radical de formule $B(R')_2$ avec R' tel que défini ci-dessus ou

- deux groupements R' peuvent être liés entre eux de manière à constituer avec l'atome de bore auquel ils sont liés un cycle à 5 ou 10 atomes avec ledit cycle pouvant être saturé, insaturé ponté et/ou aromatique et comprendre un ou plusieurs hétéroatomes choisis parmi les atomes d'oxygène, d'azote et de bore avec l'atome bore présent dans ledit cycle pouvant lui-même être substitué par un radical tel que défini pour A ou R' en formule générale I,

• les symboles A représentent indépendamment l'un de l'autre

  - un atome d'hydrogène

  - un atome d'halogène ou

  - un radical hydroxyle.

• x représente 0 ou l'entier 1 ou 2 et y l'entier 1, 2 ou 3 avec la somme de x+y étant égale à 3 et

sa ou ses formes solvatées.

Les amorceurs sont de manière générale des composés très hygroscopiques. En conséquence, ces composés peuvent se présenter sous l'aspect de mélange entre le composé tel que défini en formule générale I et sa ou ses différente(s) forme(s) hydratée(s). De même, lors de la formulation de cet amorceur avec un solvant, on observe la formation de dérivés solvatés. Ce phénomène peut s'observer avec des solvants aprotiques tels les éthers, esters et silicones ou des solvants protiques comme les alcools, acides carboxyliques, silanols, amines, thiols, l'eau ou leurs mélanges.

Les amorceurs sont particulièrement intéressants en terme de réactivité dans la mesure où ils sont actifs à faibles concentrations et ne nécessitent avantageusement que de faibles quantités d'énergie pour effectuer la réticulation. Ils sont en effet activables à une température inférieure à 150°C de préférence inférieure à 100°C voire à la température ambiante.

Les amorceurs peuvent en outre être associés à un amorceur conventionnel tel un photoamorceur cationique. Ceci est particulièrement avantageux en terme de rentabilité, dans la mesure où il s'avère ainsi possible de diminuer significativement la quantité efficace en amorceur conventionnel. La réticulation et/ou polymérisation est par ailleurs achevée totalement.

Les amorceurs utilisés se révèlent donc particulièrement avantageux en termes de rentabilité et de coût pour les procédés industriels.

Plus préférentiellement, les symboles R' de la formule générale (1) sont choisis de manière à conférer à l'atome de bore auquel ils sont liés un encombrement stérique suffisant pour lui assurer une protection efficace notamment pour prévenir son oxydation et/ou hydratation. En l'espèce, les amorceurs de formule générale (1) dans laquelle au moins un des symboles R' et de préférence au moins deux d'entre eux représentent un radical phényle ou aryle sont particulièrement intéressants.

De même, il est avantageux que les symboles R' soient substitués et notamment par des éléments et/ou

groupements électroattracteurs de manière à assurer audit atome de bore une électronégativité qui soit compatible avec ses propriétés électrophiles. C'est ainsi que s'avèrent particulièrement efficaces des amorceurs de formule générale (I) dans jaquette les symboles R' contribuent globalement avec les symboles A à un $\sigma_p$ au moins égal à celui de 3 radicaux ($C_6H_4F$).

Sont notamment préférés, les armorceurs répondant à la formule générale (Ia)

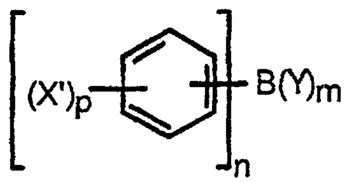

(Ia)

dans laquelle

- n représente un nombre entier compris entre 1 et 3 et m un nombre entier compris entre 0 et 2 avec la somme de n et m étant égale à 3,
- les symboles Y sont identiques ou différents et représentent

    a) un atome d'hydrogène,
    b) un groupement hydroxyle,
    c) un atome d'halogène,
    d) un radical alkyle ou alcényle en $C_1$-$C_{12}$, de préférence en $C_1$-$C_8$, linéaire ou ramifié, de préférence substitué par au moins un élément électroattracteur comme un atome d'halogène et en particulier un atome de fluor,
    e) un radical alkoxy en $C_1$-$C_{12}$, de préférence en $C_1$-$C_8$, linéaire ou ramifié, de préférence substitué par au moins un élément électroattracteur comme un atome d'halogène et en particulier un atome de fluor,
    f)-$C_2H_4$-Si(Q)$_3$ avec Q représentant un groupe alkyle ou alkoxy en $C_1$ à $C_{10}$ ou un oligomère siloxane inférieur à 10 atomes de silicium le cas échéant substitué par un radical de formule B(R')$_2$ avec R' tel que défini ci-dessus, ou
    g) deux groupements Y peuvent être liés entre eux de manière à constituer avec l'atome de bore auquel ils sont liés un cycle en $C_5$-$C_{10}$ avec ledit cycle pouvant être saturé, insaturé, ponté et/ou aromatique et comprendre un ou plusieurs hétéroatomes choisis parmi les atomes d'oxygène, d'azote et de bore avec l'atome bore présent dans ledit cycle pouvant lui-même être substitué par un radical tel que défini pour Y en formule générale (Ia) et

- les symboles X' sont identiques ou différents et représentent
- un atome d'halogène de préférence un atome de fluor,
- un radical hydrocarboné en $C_1$-$C_{12}$, de préférence en $C_1$-$C_8$, linéaire, ramifié, mono- ou poly- cyclique, saturé, insaturé ou aromatique et de préférence substitué par au moins un élément électroattracteur comme un atome d'halogène et en particulier un atome de fluor ou un radical alkyle en $C_1$-$C_{12}$, de préférence en $C_1$-$C_8$, linéaire ou ramifié, mono- poly- ou per-halogéné avec en particulier le fluor comme atome d'halogène, et
- les indices p sont identiques ou différents et représentent un entier compris entre 0 et 5, avec de préférence au moins l'un des symboles p supérieur à 3 et plus préférentiellement égal à 5.

Les amorceurs de formule générale (Ia) dans laquelle Y répond aux définitions a), b), c), d) et e) sont particulièrement intéressants.

A titre représentatif des amorceurs utilisés,on peut plus particulièrement citer les composés suivants :

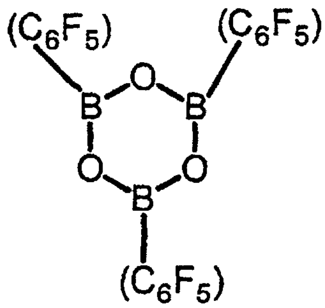 ; 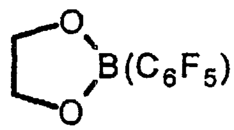 ; 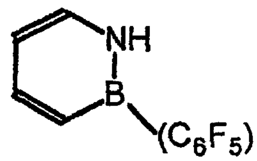

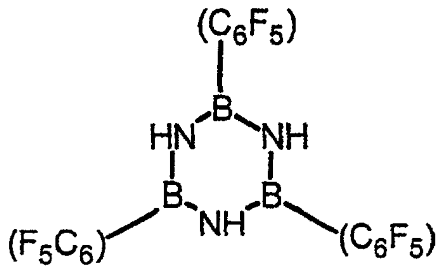 ;

$(C_5F_4)(C_6F_5)_2B$ ; $(C_5F_4)3B$ ; $(C_6F_5)BF_2$ ; $BF(C_6F_5)_2$ ; $B(C_6F_5)_3$ ; $BCl_2(C6F5)$ ; $BCl(C_6F_5)_2$ ; $B(C_6H_5)(C_6F_5)_2$ ;

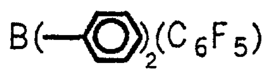 ;

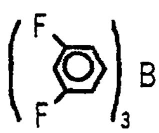 ; 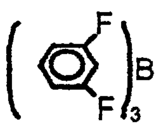 ; 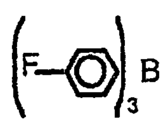 ;

$[C_6H_4(mCF_3)]_3B$ ; $[C_6H_4(pOCF_3)]_3B$ ;

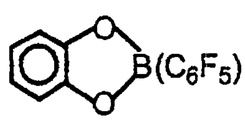 ;

$(C_6F_5)B(OH)_2$ : $(C_6F_5)_2BOH$ ; $(C_6F_5)_2BH$ ; $(C_6F_5)BH_2$ ;

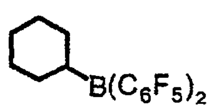 ;

$(C_7H_{11})B(C_6F_5)_2$ ; $(C_8H_{14}B)(C_6F_5)$ ;

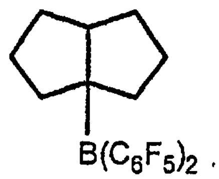 ;

$(C_6F_5)_2B(OC_2H_5)$ ;

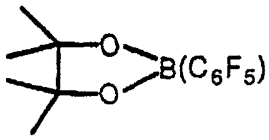 ;

$(C_6F_5)_2$ B-$CH_2$ $CH_2$ $Si(CH_3)_3$ ;

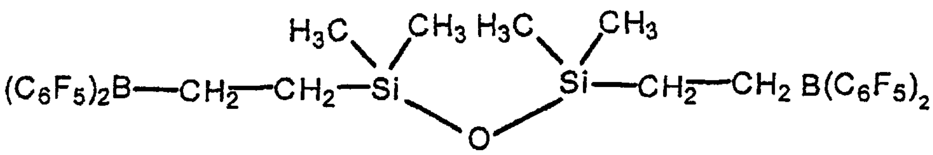 ;

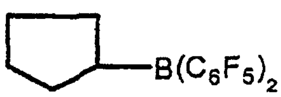 ; 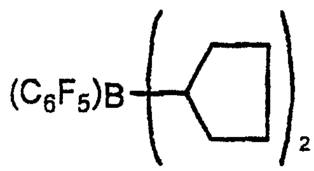 ;

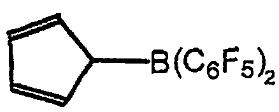 ; 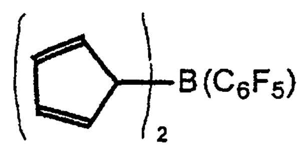

Les amorceurs peuvent être mis en oeuvre, tels qu'ils sont obtenus à l'issue de leur procédé de préparation, par exemple sous forme solide ou liquide, ou en solution dans au moins un solvant approprié, dans les compositions de monomères, oligomères et/ou polymères qui sont destinées à être polymérisées et/ou réticulées. Dans le cadre de l'invention, le terme solvant englobe les produits solubilisant les amorceurs solides et les produits diluant les amorceurs liquides ou solides.

De préférence, les amorceurs sont généralement mis en oeuvre en solution dans un solvant. Les proportions pondérales entre le ou les amorceurs, d'une part, et le solvant, d'autre part, sont comprises entre 0.1 et 99 parties pour 100 parties de solvant et, de préférence de 10 à 50 parties.

Cette solution d'amorceur est donc utilisée pour préparer un bain avec le ou les monomères, oligomères et/ou polymères à groupements fonctionnels réticulables, telle que la concentration de l'amorceur ou des amorceurs présents soit comprise entre 0.01 et 5% en poids dans ledit bain, et de préférence entre 0.05 et 0.5%.

Les solvants utilisables pour les amorceurs sont très nombreux et variés et sont choisis, selon, l'amorceur utilisé et les autres constituants de la composition de l'invention. En général, les solvants peuvent être des alcools,

des esters, des éthers, des cétones, l'eau à l'état de traces et les carbonates.

Les alcools couramment employés sont le para-tolyl-éthanol, l'isopropylbenzyl-alcool, l'alcool benzylique, le méthanol, l'éthanol, le propanol, l'isopropanol et le butanol. Les éthers communément usités sont le méthoxy-2-éthanol, l'éthoxy-2-éthanol, le diéthylène-glycol. Les esters usuels sont le dibutylmaléate, diméthyl-éthylmalonate, salycilate de méthyle, dioctyladipate, tartrate de butyle, lactate d'éthyle, lactate de n-butyle, lactate d'isopropyle. D'autres solvants utilisables pour le bain de l'amorceur et entrant dans les autres catégories de solvants citées ci-dessus sont l'acétonitrile, le benzonitrile, l'acétone, le cyclohexanone, et le tétrahydrofurane.

En outre, parmi les solvants utilisables pour mettre en solution le ou les amorceurs, certains types de solvants organiques donneurs de protons ainsi que certains types d'esters d'acides carboxyliques hydroxylés ont pour propriétés également d'améliorer significativement leurs performances de réactivité et de cinétique.

Comme signalé précédemment, en solution l'amorceur revendiqué selon l'invention peut évoluer vers une forme solvatée. Les différentes formes peuvent coexister au sein du solvant sous l'effet d'un équilibre. A titre représentatif de ces phénomènes de solvatation, on peut notamment citer

$$\text{eau } H_2O + B(Ar)_3 \rightleftarrows H^{\oplus}[B(Ar)_3(OH)]^{\ominus}$$

$$\text{alcool } R^*OH + B(Ar)_3 \rightleftarrows H^{\oplus}[B(Ar)_3(OR^*)]^{\ominus}$$

$$\text{amine } R^*_2NH + B(Ar)_3 \rightleftarrows H^{\oplus}[B(Ar)_3(NR^*_2)]^{\ominus}$$

$$\text{acide } R^*COOH + B(Ar)_3 \rightleftarrows H^{\oplus}[B(Ar)_3(OCOR^*)]^{\ominus}$$

Selon un autre de ses aspects, la présente invention concerne, dans son second objet, des compositions polymérisables et/ou réticulables comprenant au moins un monomère, un oligomère et/ou un polymère polymérisable et/ou réticulable et de nature poly-organosiloxane porteur(s) de groupements fonctionnels, une quantité efficace d'au moins un amorceur du type de ceux conformes à l'invention et décrits ci-avant, éventuellement un accélérateur de polymérisation et/ou réticulation, et éventuellement encore un ou plusieurs additifs choisis parmi ceux généralement connus dans les applications auxquelles sont destinées ces compositions.

Par quantité efficace d'amorceur, on entend selon l'invention la quantité suffisante pour amorcer la polymérisation et/ou la réticulation. Cette quantité est généralement comprise entre 0,0001 et 5 parties en poids, le plus souvent entre 0,001 et 0,5 parties en poids pour polymériser et/ou réticuler 100 parties en poids de la matière sèche en monomères, oligomères et/ou polymères polyorganosiloxane à groupements organofonctionnels.

Comme évoqué précédemment l'amorceur utilisé peut être présent dans la composition polymérisable et/ou réticulable en association avec un amorceur conventionnel tel notamment un photoamorceur cationique. Comme photoamorceurs classiques, conviennent notamment ceux décrits dans le brevet EP 562 897. Il peut également s'agir de sels d'iodonium ou de sulfonium correspondant de l' hexafluorophosphate ou hexafluoroantimonate.

Dans ce type d'association, les deux types d'amorceurs sont mis en oeuvre à raison de 0,01 à 5 parties en poids pour le photoamorceur et $1x10^{-5}$ à 5 parties en poids pour l'amorceur revendiqué, de préférence $1x10^{-4}$ à $1x10^{-2}$ pour l'amorceur revendiqué pour polymériser et/ou réticuler pour 100 parties en poids en monomère(s), oligomère(s) et/ou polymère(s) polymérisable(s) et/ou réticulable(s) de nature polyorganosiloxane porteur(s) de groupements fonctionnels.

On obtient ainsi des revêtements photoréticulables par voie cationique qui développent peu d'interactions avec les adhésifs et en particulier avec les adhésifs acryliques.

La composition polymérisable et/ou réticulable revendiquée est de préférence à base de monomère(s) et/ou oligomère(s) et/ou polymère(s), de nature polyorganosiloxane, constitués de motifs de formule (II) et terminés par des motifs de formule (III) ou cycliques constitués de motifs de formule (II) représentées ci-dessous :

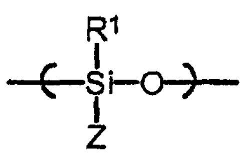
(II)

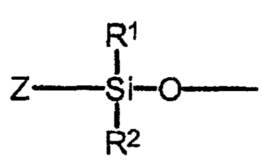
(III)

dans lesquelles :

- les symboles $R^1$ et $R^2$ sont semblables ou différents et représentent :
  - un radical alkyle linéaire ou ramifié contenant 1 à 8 atomes de carbone, éventuellement substitué par au moins un halogène, de préférence le fluor, les radicaux alkyle étant de préférence méthyle, éthyle, propyle, octyle et 3,3,3-trifluoropropyle,
  - un radical cycloalkyle contenant entre 5 et 8 atomes de carbone cycliques, éventuellement substitué,
  - un radical aryle contenant entre 6 et 12 atomes de carbone pouvant être substitué, de préférence phényle ou dichlorophényle,
  - une partie aralkyle ayant une partie alkyle contenant entre 5 et 14 atomes de carbone et une partie aryle contenant entre 6 et 12 atomes de carbone, substituée éventuellement sur la partie aryle par des halogènes, des alkyles et/ou des alkoxyles contenant 1 à 3 atomes de carbone,
- les symboles Z sont semblables ou différents et représentent :
  - un groupement $R^1$ et/ou $R^2$,
  - un radical hydrogène,
  - et/ou un groupement organofonctionnel réticulable, de préférence un groupement époxyfonctionnel, oxétanefonctionnel, dioxolanefonctionnel et/ou alcénylétherfonctionnel, relié au silicium du polyorganosiloxane par l'intermédiaire d'un radical divalent contenant de 2 à 20 atomes de carbone et pouvant contenir au moins un hétéroatome, de préférence de l'oxygène,
  - avec l'un au moins des symboles Z représentant un groupement organique fonctionnel réticulable.

Selon une variante avantageuse de l'invention, les polyorganosiloxanes utilisés comportent de 1 à 10 groupements organofonctionnels par chaine macromoléculaire. Pour un groupement époxyfonctionnel cela correspond à des taux d'époxyde variant de 20 à 2000 meq. molaire/100 g de polyorganosiloxane.

Les polyorganosiloxanes linéaires peuvent être des huiles de viscosité dynamique à 25°C, de l'ordre de 10 à 10 000 mPa.s à 25°C, généralement de l'ordre de 20 à 5 000 mPa.s à 25°C et, plus préférentiellement encore, de 20 à 600 mPa.s à 25°C, ou des gommes présentant une masse moléculaire de l'ordre de 1 000 000.

Lorsqu'il s'agit de polyorganosiloxanes cycliques, ceux-ci sont constitués de motifs (II) qui peuvent être, par exemple, du type dialkylsiloxy ou alkylarylsiloxy. Ces polyorganosiloxanes cycliques présentent une viscosité de l'ordre de 1 à 5 000 mPa.s.

Comme exemples de radicaux divalents reliant un groupement organofonctionnel du type époxy et/ou oxétane, on peut citer ceux inclus dans les formules suivantes :

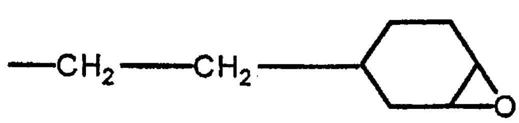 ; 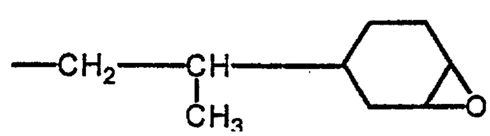

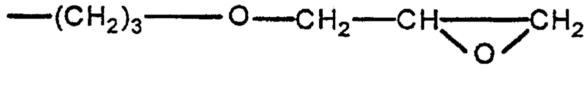 ; 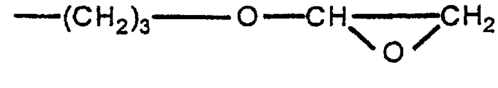

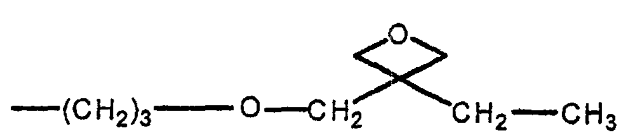

S'agissant des groupements organofonctionnels du type alcényléther, on peut mentionner ceux contenus dans les formules suivantes :

$$—(O)_{n'}—(CH_2)_{n''}—O—CH=CH_2\ ;$$

$$—(O)_{n'}—(CH_2)_{n''}—R^3—O—CH=CH_2\ ;$$

$$—(O)_{n'}—(CH_2)_{n''}—O—CH=CH—R^4$$

dans lesquelles :

- n' représente 0 ou 1 et n" un entier compris entre 1 et 5
- $R^3$ représente :
  - un radical alkylène linéaire, ramifié ou cyclique en $C_1$-$C_{12}$, éventuellement substitué, ou
  - un radical arylène en $C_5$-$C_{12}$, de préférence phénylène, éventuellement substitué, de préférence par un à trois groupements alkyles en $C_1$-$C_6$,
- $R^4$ représente un radical alkyle linéaire ou ramifié en $C_1$-$C_6$.

S'agissant des groupements dioxolane, on peut citer ceux contenus dans les formules suivantes :

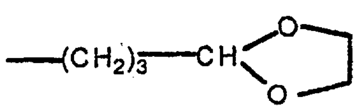 ; 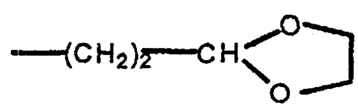 ;

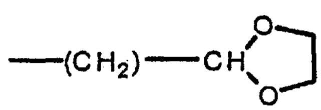

Les polyorganosiloxanes epoxy ou alcénylétherfonctionnels se présentent généralement sous forme de fluides présentant une viscosité à 25°C de 10 à 10.000 $mm^2/s$ et de préférence de 20 à 600 $mm^2/s$.

La viscosité dynamique à 25°C, de toutes les silicones considérées dans la présente description peut être mesurée à l'aide d'un viscosimètre BROOKFIELD, selon la norme AFNOR NFT 76 102 de février 1972.

Ce type de composés est décrit notamment dans les brevets DE-A- n°4.009.889 ; EP-A- n°396.130 ; EP-A- n°355,381 ; EP-A- n°105.341 ; FR-A- n°2.110.115 ; FR-A- 2.526.800.

Les polyorganosiloxanes alcénylétherfonctionnels peuvent être préparés par réaction d'hydrosilylation entre des huiles à motifs Si-H et des composés vinyloxyfonctionnels tels que l'allylvinylether, l'allylvinyloxyethoxybenzène...

Les polyorganosiloxanes époxy fonctionnels peuvent être préparés par réaction d'hydrosilylation entre des huiles à motifs Si-H et des composés époxyfonctionnels tels que vinyl-4 cyclohexeneoxyde, allylglycidyléther...

Les polyorganosiloxanes oxétane fonctionnels peuvent être préparés par hydrosilylation d'oxétanes insaturés ou condensation d'oxétanes renfermant une fonction hydroxy.

Les polyorganosiloxanes dioxolane fonctionnels peuvent être préparés par hydrosilylation de dioxolanes insaturés.

Conviennent tout particulièrement à l'invention les polyorganosiloxanes dont les motifs de formules (II) et/ou

(III) possèdent au moins un radical phényle, tolyle ou dichlorophényle à titre de radical $R^1$.

Les silicones répondant le mieux à l'objet de l'invention sont décrits ci-après et possèdent au moins un groupement époxyde, alcényléther ou oxétane.

Dans les formules ci-après X peut représenter, un groupement alkyle ; cyclohexyle ; trifluoropropyle ; perfluoroalkyle ; alcoxy ou hydroxypropyle, R un radical alkyle en $C_1$ à $C_{10}$, cyclohexyle, trifluoropropyle ou perfluoroalkyle en $C_1$ à $C_{10}$ et ($0 \leq a \leq 1000$) ; ($1 \leq b \leq 1000$)

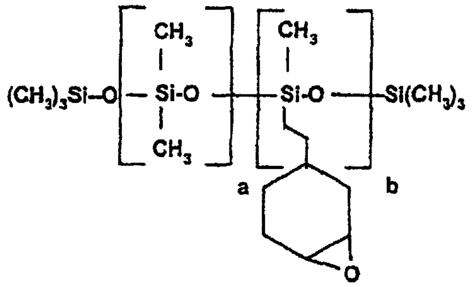

S1

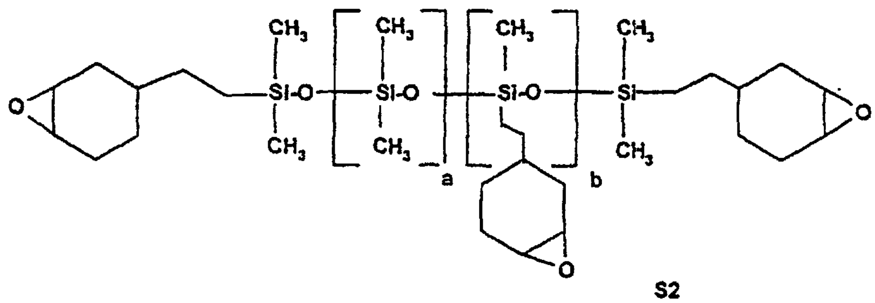

S2

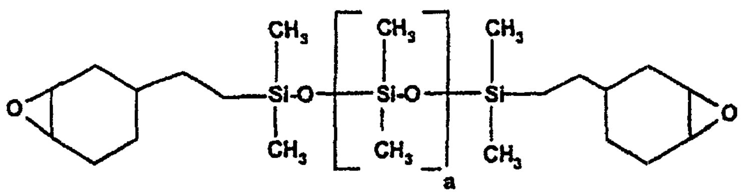

S3

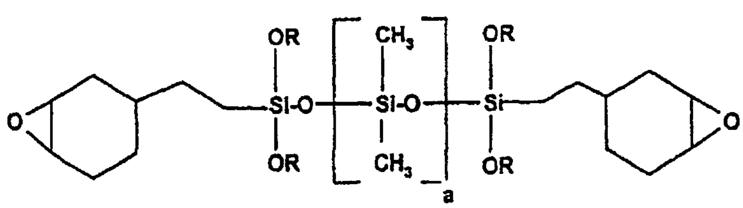

S4

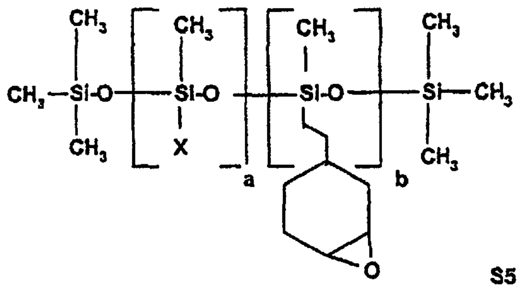

S5

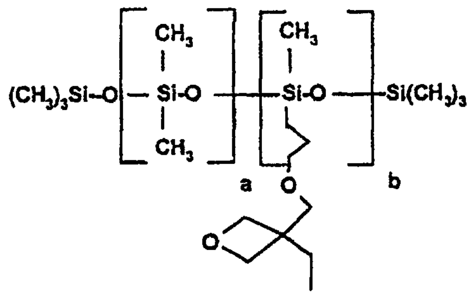

S6

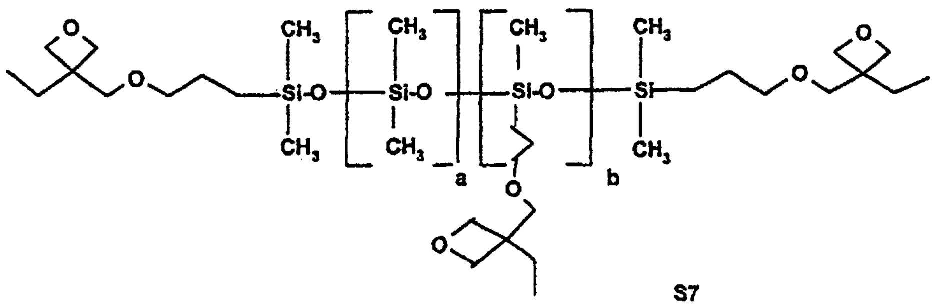

S7

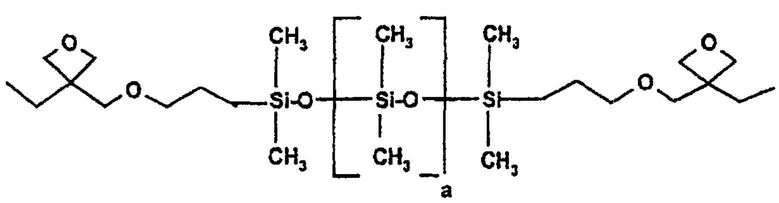

S8

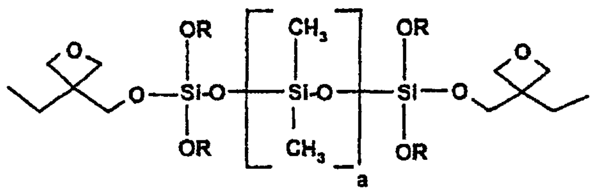

S9

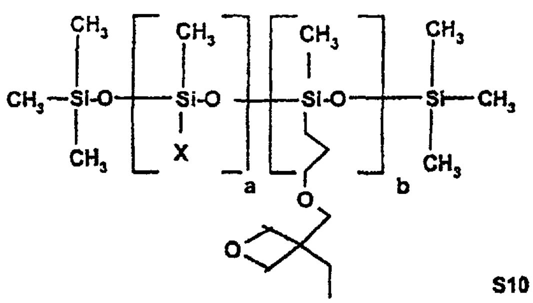

S10

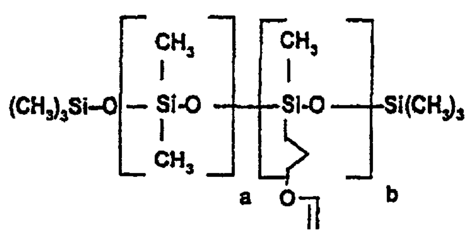

S11

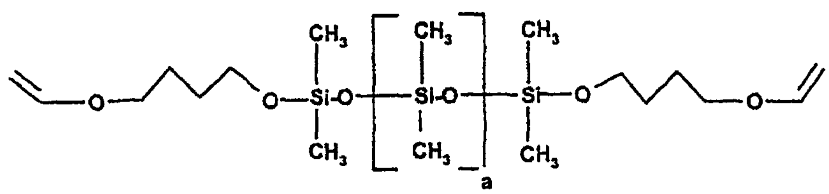

S12

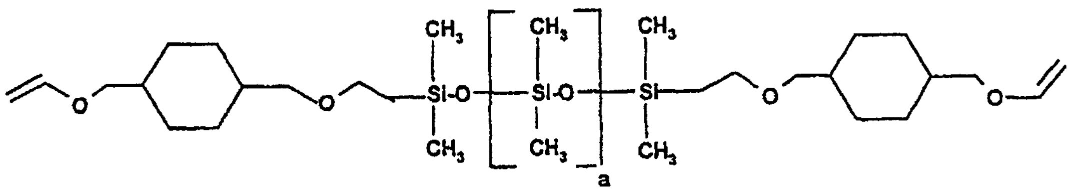

S13

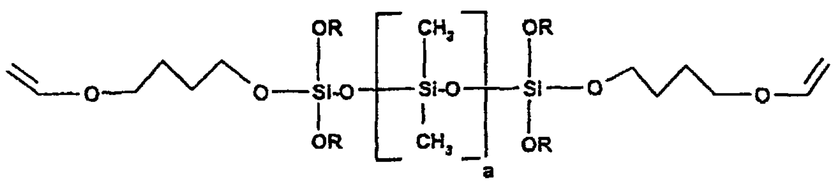

S14

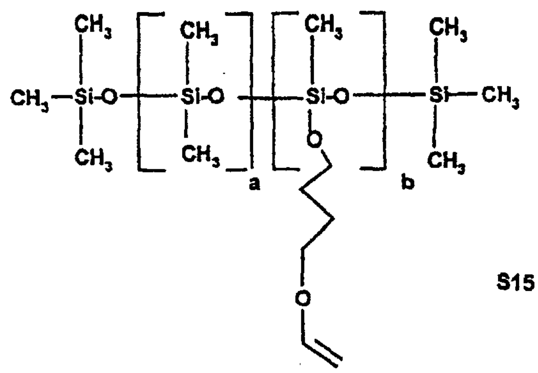

S15

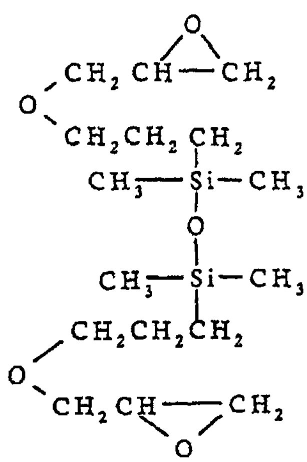

S16

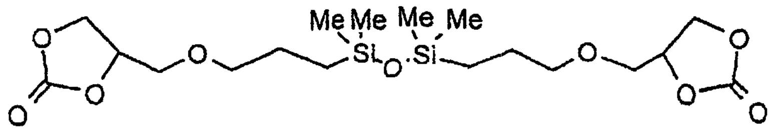

S17

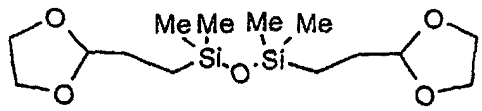

S18

Selon une disposition intéressante de l'invention prise dans son second objet, la composition polymérisable et/ou réticulable est à base de monomère(s) et/ou oligomère(s) et/ou polymère(s), de nature polyorganosiloxane tels que définis précédemment et de monomère(s), oligomère(s) ou polymère(s), de nature organique, notamment hydrocarbonée.

En l'espèce, les monomères oligomères ou polymères organiques suivants avec n pouvant varier de 0 à 1000 conviennent tout particulièrement à l'invention.

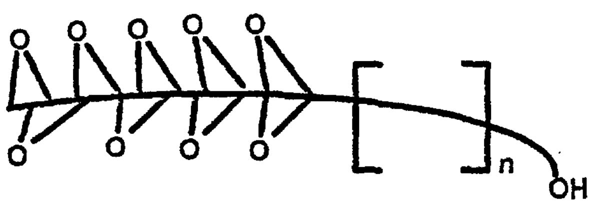

KRATON TM EKP 207 (O1)

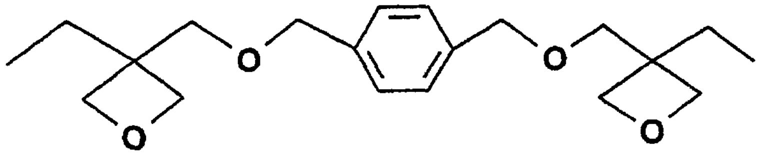

(O2)

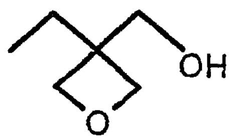

(O3)

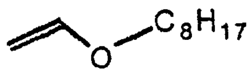

(O4)

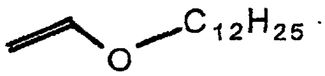

(O5)

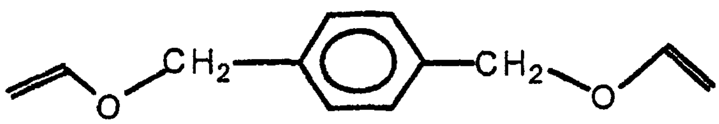

(O6)

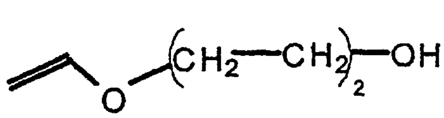

(O7)

Selon une troisième disposition intéressante de l'invention prise dans son second objet, la composition polymérisable et/ou réticulable est à base de monomère(s) et/ou oligomère(s) et/ou polymère(s), de nature polyorganosiloxane et éventuellement de nature organique, monomère(s) et/ou oligomère(s) et/ou polymère(s), notamment hydrocarbonée.

Pour la mise en oeuvre des amorceurs selon l'invention, différentes sources de chauffage peuvent être utilisées pour effectuer la polymérisation et/ou réticulation des monomères, oligomères et/ou polymères. Dans le cas particulier où l'amorceur conforme à l'invention est mis en oeuvre avec un photoamorceur cationique, la chaleur inhérente à l'irradiation utilisée pour activer ledit photoamorceur peut être suffisante pour activer simultanément l'amorceur revendiqué.

Classiquement les compositions selon l'invention peuvent comprendre, en outre, un ou plusieurs additifs choisis en fonction de l'application finale visée.

Les additifs peuvent être notamment des composés éventuellement sous forme de polymères, à hydrogènes mobiles comme des alcools, des glycols et des polyols, utiles pour améliorer la flexibilité du matériau durci après polymérisation et/ou réticulation ; on peut citer par exemple les polycaprolactones-polyols, en particulier le polymère obtenu au départ de 2-éthyl - 2-(hydroxyméthyl)-1,3-propane-diol et de 2-oxépanone tel que le produit TONE POLYOL-301 commercialisé par la Société UNION CARBIDE, ou les autres polymères commerciaux TONE POLYOL 201 et TONE POLYOL 12703 de la société UNION CARBIDE. On peut également citer comme additifs, les diacides à longue chaîne alkyle, les esters gras d'acides insaturés époxydés ou non, par exemple l'huile de soja époxydée ou l'huile de lin époxydée, le 2-éthylhexylester époxydé, le 2-éthylhexyl époxy stéarate, l'époxystéarate d'octyle, les esters acryliques époxydés, les acrylates d'huile de soja époxydés, les acrylates d'huile de lin époxydés, l'éther, diglycidique de glycolpolypropylène, les époxydes aliphatiques à longue chaîne; etc..

Cet additif peut être notamment un additif de stabilisation. Il s'agit généralement d'un agent aminé comporte au moins une amine dont le point d'ébullition est supérieur à 150°C et de préférence supérieur à 200°C. Cette amine

peut être une amine secondaire ou une amine tertiaire.

On peut notamment utiliser les amines décrits dans WO 98/07798.

Il est à noter que la plupart des amines encombrées utilisées comme agent de stabilité à la lumière (type "HALS") se révèlent être de très bonnes candidates pour satisfaire aux exigences des agents stabilisants utilisés dans le cadre de l'invention, bien que leur propriété intrinsèque de stabilité à la lumière n'ait pas de relation directe avec le mode d'action des agents aminés stabilisants des compositions selon l'invention. A ce sujet, il est possible d'utiliser les différents types d'amines encombrées des documents EP 162 524 et EP 263 561.

De nombreux types d'amines encombrées disponibles dans l'industrie ont donné de bons résultats, et notamment :

- les produits TINUVIN commercialisés par la société CIBA GEIGY, en particulier les produits TINUVIN 144 et TINUVIN 765, décrits ci-après,
- les produits CYAGARD commercialisés par CYTEC, en particulier le produit CYAGARD UV 1164L, et
- les produits SANDUVAR, en particulier le produit SANDUVAR 3055, décrit ci-après commercialisé par la société SANDOZ.

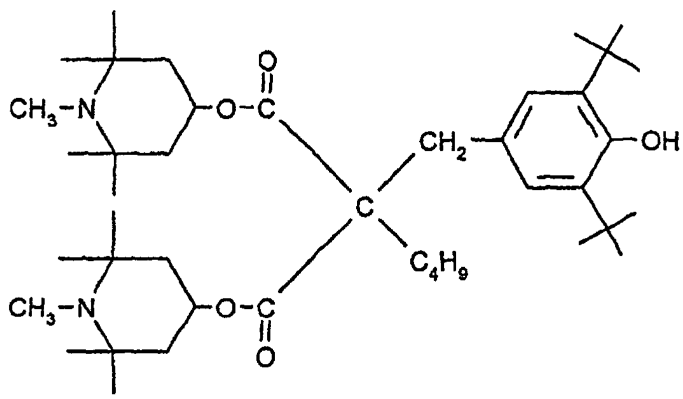

Tinuvin 144

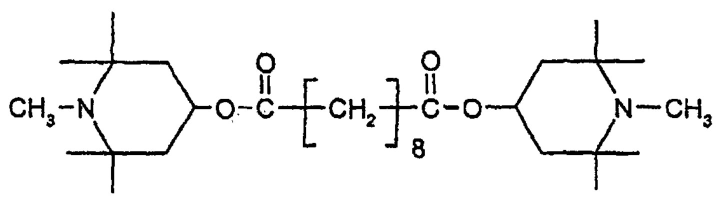

Tinuvin 765

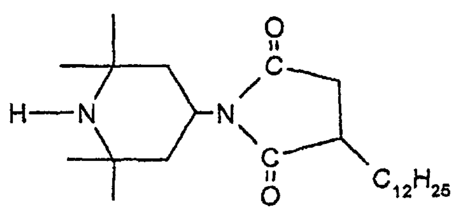

Sanduvar 3055

D'autres types d'amines répondant aux formules suivantes sont également de bonnes candidates pour être utilisées dans les compositions de l'invention; à titre d'exemple, la structure de certaines de ces amines est donnée ci-dessous :

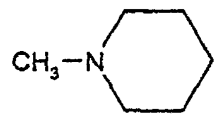

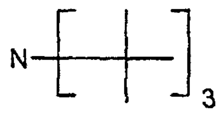

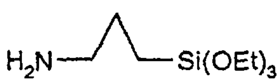 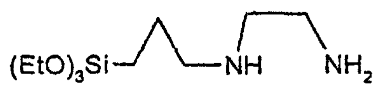

Le pourcentage d'agent aminé généralement utilisé en poids par rapport au poids total de la matrice silicone est compris entre 1 et 1.000 ppm et de préférence entre 10 et 100 ppm. Dans le cas d'agent aminé de type HALS, la quantité est de l'ordre de 20 à100 ppm.

Les compositions selon l'invention peuvent comporter en outre d'autres ingrédients tels que des modulateurs d'adhérence permettant d'augmenter ou de diminuer les forces d'adhérence obtenues à partir du polyorganosiloxane seul (résines ou polymères linéaires silicones portant des fonctions vinyle, époxy, vinyléther, alcool), des pigments, des photosensibilisateurs, des agents fongicides, bactéricides et anti-microbiens, des inhibiteurs de corrosion, etc..

Il peut s'agir en outre, quelle que soit la nature de la matrice polymérisable, par exemple : de charges telles que notamment des fibres synthétiques du dioxyde de titane, de la silice de précipitation ou de combustion ; de colorants solubles ; d'inhibiteurs d'oxydation et de corrosion ; de modulateurs d'adhérence organosiliciques ou non ; d'agents fongicides, bactéricides, anti-microbiens ; et/ou de tout autre matériau n'interférant pas avec l'activité de l'amorceur.

La présente invention a également pour objet les résines ou polymères susceptibles d'être obtenus à partir des compositions décrites précédemment.

Dans le cas particulier où les compositions sont utilisées pour préparer des compositions dentaires, différents types de charges peuvent être utilisés. Les charges sont choisies en fonction de l'utilisation finale de la composition dentaire : celles-ci affectent d'importantes propriétés telles que l'apparence, la pénétration du rayonnement U.V. , ainsi que les propriétés mécaniques et physiques du matériau obtenu après réticulation et/ou polymérisation de la composition dentaire.

Comme charge de renforcement, on peut utiliser des charges de silice de pyrogénation traitée ou non, des charges de silice amorphe, du quartz, des verres ou des charges non vitreuses à base d'oxydes de zirconium, de baryum, de calcium, de fluor, d'aluminium, de titane, de zinc, des borosilicates, des aluminosilicates, du talc, des sphérosil, du trifluorure d'yterbium, des charges à base de polymères sous forme de poudre broyée tel que des polyméthacrylates de méthyle inertes ou fonctionnalisés, des polyépoxydes ou des polycarbonates.

A titre d'exemple, on citera :

- des charges inertes à base de polyméthacrylate de méthyle LUXASELF de la société UGL utilisables dans le domaine dentaire et pigmentées en rose,
- des charges de silice de combustion traitée hexaméthyldisilazane de surface spécifique 200 $m^2$/g,
- des charges de silice de combustion non traitée (« aerosil » AE200 commercialisée par DEGUSSA ).

Les charges et en particulier les charges de silice peuvent être traitées avant utilisation à 120°C avec une quantité inférieure à 10% p/p de silicone comprenant au moins un motif de formule :

$$P-Si-(R^5)_{a'}-O_{(3-a')/2}$$

- avec P, identique ou différent, étant un substituant organique comprenant au moins une fonction réactive époxy,

et/ou alcényléther et/ou oxétane et/ou dioxolane et/ou carbonate,

- $R^5$, identique ou différent, représentant un radical alkyle, cycloalkyle, aryle, vinyle, hydrogéno, alcoxy, de préférence un alkyle inférieur en $C_1$-$C_8$,
- a'=0,1 ,2 ou 3,
- avec au moins un atome de silicium.

On peut citer à titre d'exemple, le polymère décrit ci-dessous avec P= époxyde et P= trialcoxysilyle

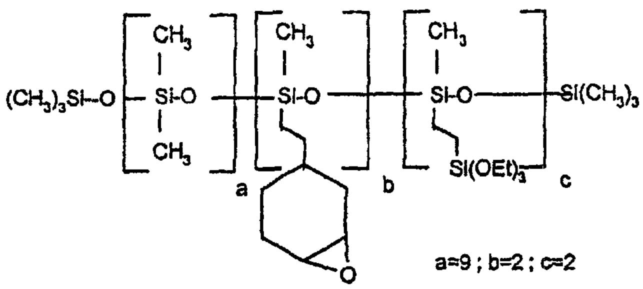

Les compositions dentaires selon l'invention peuvent être utilisées pour de nombreuses applications dentaires, et en particulier dans le domaine des prothèses dentaires, dans le domaine de la restauration dentaire et dans le domaine des dents provisoires.

Avantageusement, en application dentaire, les compositions conformes à l'invention permettent une prise rapide à une température ambiante et une réduction significative du phénomène de retrait usuellement noté avec des compositions de silicone classiques.

Dans le domaine des prothèses dentaires, la composition dentaire selon l'invention se présente de préférence sous la forme d'un seul produit contenant les différents composants ("*monocomposant*") ce qui facilite sa mise en oeuvre. Eventuellement, la stabilité de ce produit peut être assurée par des dérivés organiques à fonctions amines selon l'enseignement du document WO 98/07798.

Le produit peut être déposé à l'aide d'une seringue directement sur le modèle en plâtre ou dans une clé. Puis, il est polymérisé (polymérisation par couches successives possibles) à l'aide d'une lampe UV (spectre lumière visible 200 - 500 nm). En géneral, la réalisation en 10 à 15 min une prothèse dentaire esthétique.

Il est à noter que les produits obtenus à partir de la composition dentaire selon l'invention sont non poreux. Ainsi, après éventuellement polissage à l'aide d'une brosse feutre par exemple, la surface des prothèses dentaires obtenues est lisse et brillante et donc ne nécessite pas d'utilisation de vernis.

Les applications dans le domaine des prothèses dentaires sont essentiellement celles de la prothèse adjointe.

Dans le domaine de la restauration dentaire, la composition dentaire selon l'invention peut être utilisée en tant que matériau d'obturation des dents antérieures et postérieures en différentes teintes (par exemple, teintes 'VITA"), rapide et facile à mettre en oeuvre.

La composition dentaire étant non toxique et polymérisable en couches épaisses, il n'est pas indispensable de polymériser le matériau en couches successives. En général, une seule injection de la composition dentaire est suffisante.

Les préparations pour prothèses dentaires et pour matériaux de restauration sont effectuées selon les techniques usuelles du métier.

Pour ce qui est des autres applications, les compositions selon l'invention sont utilisables telles quelles ou en solution dans un solvant organique. Elles sont utiles dans le domaine des revêtements anti-adhérents sur les matériaux cellulosiques, les peintures, l'encapsulation de composants électriques et électroniques, les revêtements pour textiles, ainsi que pour le gainage de fibres optiques.

Elles sont tout particulièrement intéressantes lorsqu'elles sont utilisées telles quelles pour rendre un matériau non adhérent, tel que des feuilles métalliques, du verre, des matières plastiques ou du papier, à d'autres matériaux auxquels il adhéreraient normalement. La composition présente avantageusement une viscosité ne dépassant pas 5 000 mPa.s, de préférence ne dépassant pas 4 000 mPa.s à 25°C.

L'invention vise donc également un procédé permettant de rendre des articles (feuilles par exemple) non adhérents à des surfaces auxquelles ils adhèrent normalement, procédé caractérisé en ce qu'il consiste à appliquer une quantité de composition de l'invention, comprise généralement entre 0,1 et 5 g par $m^2$ de surface à enduire et à réticuler ou polymériser la composition en l'exposant à une source de chauffage.

Cette invention s'étend également aux revêtements dérivant des compositions résines et/ou polymères revendiqués. Il peut s'agir de revêtement de type vernis, revêtement adhésif, revêtement anti-adhérent et/ou une encre. On peut également obtenir des revêtements silicones dans le domaine de l'encapsulation de composants électroniques ou de revêtements pour fibres optiques.

Les compositions sans solvant, c'est-à-dire non diluées, sont appliquées à l'aide de dispositifs aptes à déposer, d'une façon uniforme, de faibles quantités de liquides. On peut utiliser à cet effet par exemple le dispositif nommé "Helio glissant" comportant en particulier deux cylindres superposés : le rôle du cylindre placé le plus bas, plongeant dans le bac d'enduction où se trouve la composition, est d'imprégner en une couche très mince le cylindre placé le plus haut, le rôle de ce dernier est alors de déposer sur le papier les quantités désirées de composition dont il est imprégné, un tel dosage est obtenu par réglage de la vitesse respective des deux cylindres qui tournent en sens inverse l'un de l'autre.

Les quantités de compositions déposées sur les supports sont variables et s'échelonnent le plus souvent entre 0,1 et 5 $g/m^2$ de surface traitée. Ces quantités dépendent de la nature des supports et des propriétés antiadhérentes recherchées. Elles sont plus souvent comprises entre 0,5 et 1,5 $g/m^2$ pour des supports non poreux.

La présente invention vise également l'utilisation d'un composé de formule générale I telle que définie ci-dessus à titre d'amorceur de polymérisation et/ou de réticulation activable thermiquement, pour des monomères, oligomères et/ou de polymères de nature polyorganosiloxane à groupements organofonctionnels notamment tels que définis précédemment.

La présente invention a également pour objet les articles (feuilles par exemple) constituées d'un matériau solide (métal, verre, matière plastique, papier ...) dont une surface au moins est revêtue de la composition ci-dessus réticulée thermiquement.

Les exemples suivants sont donnés à titre illustratif et ne peuvent être considérés comme une limite du domaine et de l'esprit de l'invention.

EXEMPLE 1

On utilise le polymère (S1)

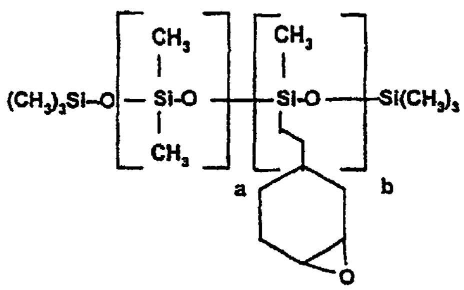

S1

à raison de 10g tel que a=80 ;b=7.

Ce polymère est stabilisé ou non par 50 ppm d'une amine encombrée utilisée comme agent de stabilité à la lumière à savoir le TINUVIN 765®.

On rajoute l'amorceur $(C_6F_5)_3$ B dit P1 à 18% en solution dans le dibutyléther.

La concentration du trispentafluorophenylborane est de 0,18% dans le silicone.

On enregistre le temps de prise en masse du polymère silicone après mélange sous agitation. Le temps de gel correspondant au passage de l'état liquide à l'état solide est inférieur à 30 secondes à 25°C et trouvé égal à 10s.

EXEMPLE 2

On réalise la même expérience qu'à l'exemple 1 en rajoutant de l'amorceur P1 dans l'éther butylique à 9% soit 0,09% dans le silicone.

Le temps de gel est trouvé égal à 13 secondes.

EXEMPLE 3

On réalise la même expérience qu'à l'exemple 1 en introduisant l'amorceur P1 dans l'isopropanol à 9% soit 0,08% dans le silicone.

On enregistre le temps de gel qui est de trois minutes à température ambiante.

EXEMPLE 4

On réalise la même réaction qu'à l'exemple 1 mais en introduisant l'amorceur P1 à raison de 270 ppm dans le polymère (S1) sous forme d'une solution dans l'éther butylique à 18%. Le mélange est appliqué en couche mince à l'aide d'une barre calibrée Barre meyer 0 de façon à déposer 1,5 à 2g/$m^2$sur un papier couché (Lohjan).

La durée de vie en pot sous agitation est de 1 minute.

Le revêtement polymérise en moins de 30s à 100°C conduisant à une couche réticulée qui présente du Rub-Off. (gommage au toucher).

Le revêtement polymérisé en une minute à 100°C conduit à une couche réticulée qui ne présente aucun Rub-Off et semble parfaitement polymérisée.

Les couches polymérisées obtenues sont ensuite adhésivées avec un adhésif acrylique du type TESA4970®. Les complexes sont mis en pression à 70g /$cm^2$ et les forces de décollement sont mesurées après 20h à 70°C selon le test FINAT n°10 et 7 jours à 70°C.

Les forces de décollement obtenues par pelage à 180° sur un dynamomètre sont inférieures à 20g/cm.

EXEMPLE 5

On réalise la même réaction qu'à l'exemple 1 mais en introduisant l'amorceur P1 à raison de 900 ppm dans le polymère (S1) sous forme d'une solution dans l'isopropanol à 9%. Le mélange est appliqué en couche mince à l'aide d'une barre calibrée Barre meyer 0 de façon à déposer 1,5 à 2g/$m^2$sur un papier couché Lohjan.

La durée de vie en pot sous agitation est de 3 minutes.

Le revêtement polymérise en moins de 10s à 100°C conduisant à une couche réticulée qui ne présente aucun Rub-Off et semble parfaitement polymérisée.

Les couches polymérisées obtenues sont ensuite adhésivées à 15 minutes avec un adhésif acrylique du type TESA4970. Les complexes sont mis en pression à 70g/$m^2$ et les forces de décollement sont mesurées après 20h à 70°C.

Les forces de décollement obtenues par pelage à 180° de l'adhésif sur un dynamomètre sont inférieures à 50 cN/inch.

EXEMPLES 6 A 15

Ces exemples montrent que l'association d'un trisarylborane à un photoamorceur cationique à base de sel de diaryliodonium ou de triarylsulfonium permet de gagner en réactivité et conduisent à une polymérisation complète.

- L'amorceur P1 utilisé est :

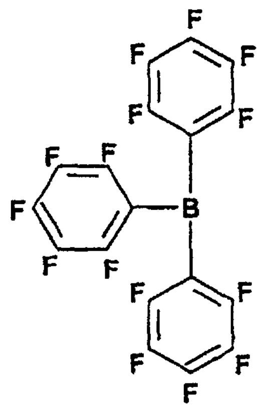

P1

- Le photoamorceur PI utilisé correspond à la formule :

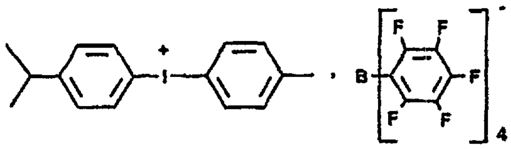

Le polymère silicone utilisé répond à la formule générale :

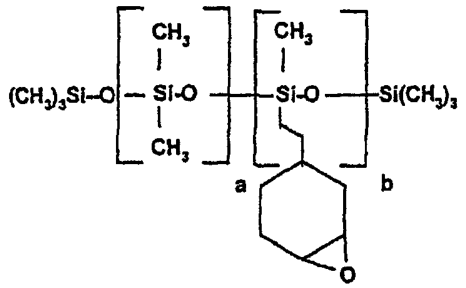

S1

Dans le cas des exemples 6 à 10 :

On utilise le polymère (S1a) à raison de 950 g tel que a=80 ;b=7
Ce polymère est stabilisé par 50 ppm de Hals (TINUVIN 765 )
On utilise également un polymère (S1b) à raison de 50g tel que a=220 ; b=3,8
On rajoute l'amorceur P1 à raison de 0 , 10 , 25, 50 ou 100 ppm dans les deux polymères S1a et S1b à partir de cinq solutions de photoamorceur PI à 18% dans l'isopropanol additivée d'amorceur P1 .

Exp 6 :Solution1 à 0% de P1 et 18% de PI : On ajoute 25 g de solution1

Exp 7 :Solution2 à 0,04% de P1 et 18% de PI . On ajoute 25 g de solution2

Exp 8 :Solution3 à 0,1 % de P1 et 18% de PI . On ajoute 25 g de solution3

Exp 9 :Solution4 à 0,2% de P1 et 18% de PI . On ajoute 25 g de solution4

Exp 10 :Solution5 à 0,4% de P1 et 18% de PI . On ajoute 25 g de solutions

On mélange les amorceurs dans la formulation silicone à l'aide d'un mélangeur Tripale pendant une demi-heure à température ambiante. Le bain est stable pendant plus de 24 heures.
On applique ensuite le silicone sur un film polyester téréphtalate à l'aide de rouleaux enducteurs de telle façon que le revêtement, représente une couche de $1 g/m^2$.
La vitesse de défilement du film est de 100 m/min .
Le film est exposé sous une lampe UV de puissance 120W/cm et on applique immédiatement après exposition un adhésif à base d'acrylate de butyle en émulsion de la gamme RHODOTAK (RHODOTAK315P®) . L'adhésif est séché à 110°C dans un four soit à vitesse de défilement du film (100 m/min) « in-line » soit indépendamment sur une autre ligne « off line » à une vitesse de défilement beaucoup moins rapide.
Après séchage de l'adhésif à la surface du revêtement silicone on transfère l'adhésif sur un papier velin à l'aide d'un rouleau de transfert. On obtient alors un complexe auto-adhésif dont on mesure les forces de décollement au cours du temps après mise en pression des étiquettes sous $70 g/cm^2$. Le test est normalisé et correspond aux normes FINAT.

FINAT3 = 20hà 20°C

FINAT10=20hà70°C qui simule un vieillissement naturel de 3 mois à 20°C.

Dans le cas des exemples 11 à 15 :

On réalise les mêmes revêtements que pour les exemples 6 à 10 excepté le dépôt silicone qui est de 1,5g/$m^2$ pour un support de rugosité plus élevée à base de papier couché (Lopabase w67) de la société Lohjan .
Les mêmes mesures des forces de décollement sont effectuées dans les exemples 11 à 15.
Les résultats sont exprimés en cN/inch et présentés dans le tableau 1.

TABLEAU I

| Exemple | PI(ppm) | P1 (ppm) | F3 cN/inch | F10 cN/inch |
|---|---|---|---|---|
| 6 | 4300 | 0 | 5,08 | 61 |
| 7 | 4300 | 10 | 4,31 | 55,9 |
| 8 | 4300 | 25 | 4,57 | 50,8 |
| 9 | 4300 | 50 | 7,7 | 40,6 |
| 10 | 4300 | 100 | 5,08 | 30,5 |
| 11 | 4300 | 0 | 7,62 | 155 |
| 12 | 4300 | 10 | 4,57 | 88,9 |
| 13 | 4300 | 25 | 7,62 | 96,5 |
| 14 | 4300 | 50 | 5,08 | 83,8 |
| 15 | 4300 | 100 | 0,35 | 55,9 |

De plus, les forces de décollement évoluent moins avec ce type d'adhésif réputé des plus agressif et appliqué en phase aqueuse. On obtient ainsi des revêtements photoréticulables par voie cationique qui développent peu d'interactions avec les adhésifs et en particulier avec les adhésifs acryliques. La force de décollement est d'autant plus faible que l'on rajoute l'amorceur 1.

EXEMPLE 16

On reproduit l'exemple 10 en diminuant par deux la quantité de photoamorceur PI et en gardant 100 ppm de trisarylborane dans le mélange final.
On trouve après mesure des forces de décollement des valeurs équivalente à l'essai 6 sans Borane. Le gain en photoamorceur est donc multiplié par deux.

TABLEAU II

| Exemple | F3 cN/inch | F10 cN/inch |
|---|---|---|
| 16 | 5,08 | 55,9 |

**Revendications**

1. Utilisation à titre d'amorceur, pour la polymérisation et/ou de réticulation activable thermiquement, de monomères, oligomères et/ou de polymères de nature polyorganosiloxane à groupements organofonctionnels, d'au moins un dérivé du bore de formule (I).

$$(A)_xB(R')_y \qquad (I)$$

Dans laquelle

- les symboles R' sont identiques ou différents et représentent

  - un radical alkyle ou alcényle en $C_1$-$C_{12}$, de préférence en $C_1$-$C_8$, linéaire ou ramifié, éventuellement substitué par au moins un élément électroattracteur, notamment un atome d'halogène ou un groupement étectroattracteur.

- un radical alkoxy en $C_1$-$C_{12}$, de préférence en $C_1$-$C_8$, linéaire ou ramifié, éventuellement substitué par au moins un élément électroattracteur, notamment un atome d'halogène ou un groupement électroattracteur,
- un radical phényle substitué par au moins un élément électroattracteur, notamment un atome d'halogene ou un groupement électroattracteur,
- un radical aryle contenant au moins deux noyaux aromatiques tel que biphényle, naphtyle, éventuellement substitué par au moins un élément électroattracteur, notamment un atome d'halogène ou un groupement électroattracteur,
- un radical $-C_2H_4Si(Q)_3$ avec les symboles Q identiques ou différents représentant un groupe alkyle ou alkoxy en $C_1$ à $C_{10}$ ou un oligomère siloxane inférieur à 10 atomes de sillicium, le cas échéant substitué par un radical de formule $B(R')_2$ avec R' tel que défini ci-dessus ou
- deux groupements R' peuvent être liés entre eux de manière à constituer avec l'atome de bore auquel ils sont liés un cycle à 5 ou 10 atomes avec ledit cycle pouvant être saturé, insaturé, ponté ou aromatique et comprendre un ou plusieurs hétéroatomes choisis parmi les atomes d'oxygène, d'azote et de bore avec l'atome bore présent dans ledit cycle pouvant lui-mérne être substitué par un radical tel que défini pour A ou R' en formule générale I.
- les symboles A représentent indépendamment l'un de l'autre
    - un atome d'hydrogène
    - un atome d'halogène ou
    - un radical hydroxyle.
- x représente 0 ou l'entier 1 ou 2 et y un entier 1,2 ou 3 avec la somme de n+y étant égale à 3 et

sa ou ses formes solvatées.

**2.** Utilisation selon la revendication 1, **caractérisée en ce que** les symboles R' sont choisis de manière à conférer à l'atome de bore auquel ils sont liés un encombrement stérique suffisant pour lui assurer une protection efficace contre des phénomènes d'oxydation et/ou d'hydratation.

**3.** Utilisation selon la revendication 1 ou 2, **caractérisée en ce que** les symboles R' contribuent globalement avec les symboles A à un $\sigma_p$ au moins égal à celui de 3 radicaux $(C_6H_4F)$.

**4.** Utlisationr selon l'une des revendications 1 à 3, **caractérisée en ce que** ledit amorceur répond à la formule générale (la)

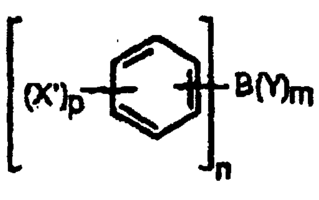

(la)

Dans laquelle

- n (représente un nombre entier compris entre 1 et 3 et m un nombre entier compris entre 0 et 2 avec la somme de n et m étant égale à 3,
- les symboles Y sont identiques ou différents et représentent

  - un atome d'hydrogène,
  - un groupement hydroxyle,
  - un atome d'halogène,
  - un radical alkyle ou alcényle en $C_1$-$C_{12}$, de préférence en $C_1$-$C_8$, linéaire ou ramifié, de préférence substitué par au moins un élément atome d'halogène,
  - un radical alkoxy en $C_1$-$C_{12}$, de préférence en $C_1$-$C_8$, linéaire ou ramifié, de préférence substitué par au moins un élément atome d'halogène,
  - -$C_2H_4$-$Si(Q)_3$ avec Q représentant un groupe alkyle ou alkoxy en $C_1$ à $C_{10}$ ou un oligomère siloxane inférieur à 10 atomes de silicium le cas échéant substitué par un radical de formule $B(R')_2$ avec R' tel que défini ci-dessus, ou
  - deux groupements Y peuvent être liés entre eux de manière à constituer avec l'atome de bore auquel ils sont liés un cycle en $C_5$-$C_{10}$ avec ledit cycle pouvant être saturé, insaturé, ponté ou aromatique et comprendre un ou plusieurs hétéroatomes choisis parmi les atomes d'oxygène, d'azote et de bore avec l'atome bore présent dans ledit cycle pouvant lui-même être substitué par un radical tel que défini pour Y en formule générale (la) et

- les symboles X' sont identiques ou différents et représentent

  - un atome d'halogène,

  - un radical hydrocarboné en $C_1$-$C_{12}$, de préférence en $C_1$-$C_8$, linéaire, ramifié, mono- ou poly- cyclique, saturé, insaturé ou aromatique et de préférence substitué par au moins un élément atome d'halogène

  - ou un radical alkyle en $C_1$-$C_{12}$, linéaire ou ramifié, mono- poly- ou per-halogéné, et

- les indices p sont identiques ou différents et représentent un entier compris entre 0 et 5, avec de préférence au moins l'un des symboles p supérieur à 3 et plus préférentiellement égal à 5.

**5.** Utilisation selon l'une des revendications 1 à 4, **caractérisée en ce que** ledit amorceur est choisi parmi les composés suivants :

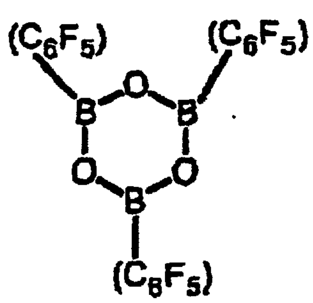

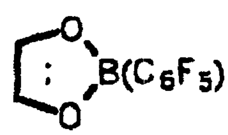

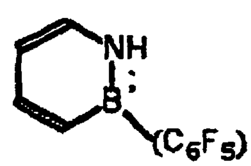

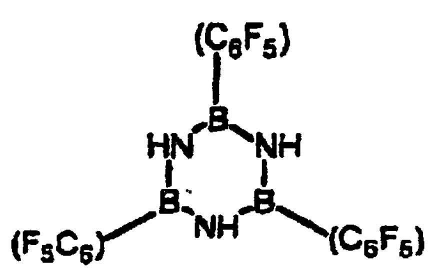

;

$(C_5F_4)(C_6F_5)_2B$ ; $(C_5F_4)_3B$ ; $(C_6F_5)BF_2$ ; $BF(C_6F_5)_2$; $B(C_6F_5)_3$ ; $BCl_2(C_6F_5)$ ;$BCl(C_6F_5)_2$ ; $B(C_6H_5)(C_6F_5)_2$ ;

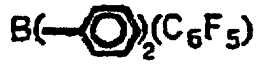 ;

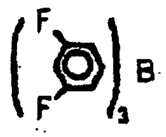 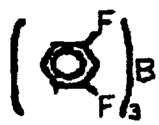 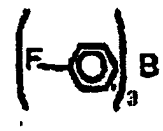 ;

$[C_6H_4(mCF_3)]_3B$ ; $(C_6H_4(pOCF_3)]_3B$ ;

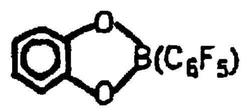 ;

$(C_6F_5)B(OH)_2$ ; $(C_6F_5)_2BOH$ ; $(C_6F_5)_2BH$ ; $(C_6F_5)BH_2$ ;

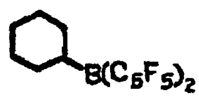 ;

$(C_7H_{11})B(C_6F_5)_2$ ; $(C_8H_{14}B)(C_6F_5)$ ;

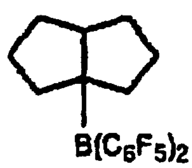 ; $(C_6F_5)_2B(OC_2H_5)$ 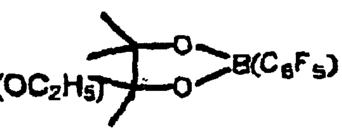 ;

$(C_6F_5)_2$ B-$CH_2$ $CH_2$ $Si(CH_3)_3$ ;

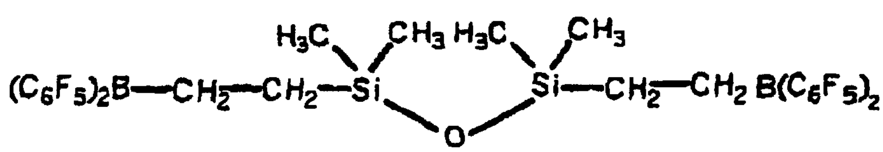

;

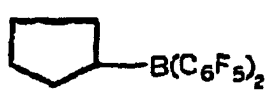 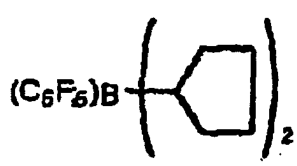 ;

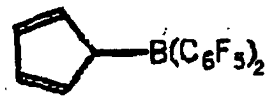
:
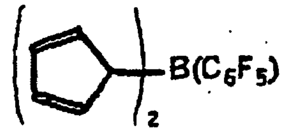

**6.** Utilisation selon l'une des revendications précédentes, **caractérisée en ce que** ledit amorceur est utilisé en solution dans un solvant.

**7.** Composition polymérisable et/ou réticulable **caractérisé en ce que** la composition comprend au moins un monomère, oligomère et/ou polymère polyorganosiloxane à groupements organofonctionnels et une quantité efficace d'au moins un amorceur activable thermiquement selon l'une quelconque des revendications précédentes.

**8.** Composition selon la revendication 7, **caractérisé en ce que** l'amorceur est utilisé à raison de 0,0001 à 5 parties en poids pour 100 parties en poids de la matière sèche en monomères, oligomères et/ou polymères polyorganosiloxane à groupements organofonctionnels.

**9.** Compositlon selon la revendication 7 ou 8, **caractérisée en ce que** les monomères, oligomères et/ou polymères de nature polyorganosiloxane possèdent à titre de groupements organofonctionnels des groupements époxydes, oxétanes, dioxolanes et/ou alcényléthers.

**10.** Composition selon la revendication 7. 8 ou 9, **caractérisée en ce que** les monomères, oligomères et/ou polymères polyorganosiloxane à groupements organofonctionnels sont constitués de motifs de formule (II) et terminés par des motifs de formule (III) ou cycliques constitués de motifs de formule (II) représentées ci-dessous :

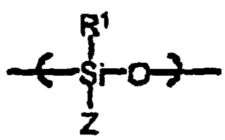 (II)

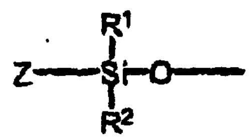 (III)

dans lesquelles :

- les symboles $R^1$ et $R^2$ sont semblables ou différents et représentent :
    - un radical alkyle linéaire ou ramifié contenant 1 à 8 atomes de carbone, éventuellement substitué par au moins un halogène,
    - un radical cycloalkyle contenant entre 5 et 8 atomes de carbone cycliques, éventuellement substitué,
    - un radical aryle contenant entre 6 et 12 atomes de carbone pouvant être substitué,
    - une partie aralkyle ayant une partie alkyle contenant entre 5 et 14 atomes de carbone et une partie aryle contenant entre 6 et 12 atomes de carbone, substituée éventuellement sur ta partie aryle par des halogènes, des alkyles et/ou des alkoxyles contenant 1 à 3 atomes de carbone,
- les symboles Z sont semblables ou différents et représentent :
    - un groupement $R^1$ et/ou $R^2$,
    - un radical hydrogène,
    - et/ou un groupement organofonctionnel réticulable, de préférence un groupement époxyfonctionnel, oxétanefonctionnel, dioxolanefonctionnel et/ou alcénylétherfonctionnel, relié au silicium du polyorganosiloxane par l'intermédiaire d'un radical divalent contenant de 2 à 20 atomes de carbone et pouvant contenir

au moins un hétéroatome,

- avec l'un au moins des symboles Z représentant un groupement organique fonctionnel réticulable.

**11.** Composition selon la revendication 10, **caractérisée en ce que** les groupements organofonctionnels sont choisis parmi :

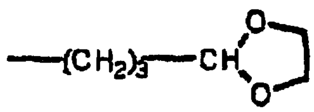 ; 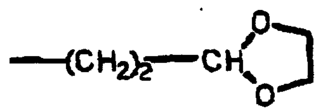

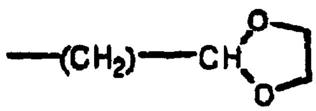

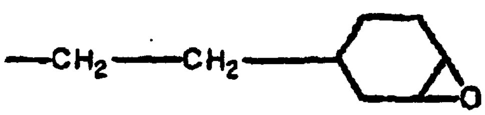 ; 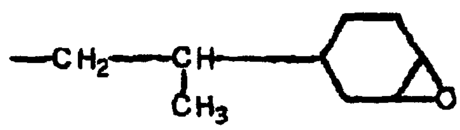

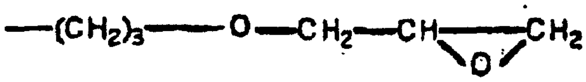 ; 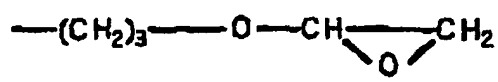

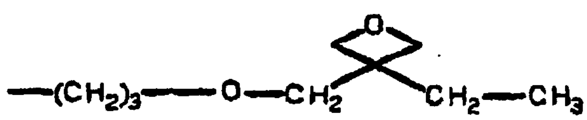

$$-(O)_{n'}-(CH_2)_{n''}-O-CH=CH_2 \; ;$$

$$-(O)_{n'}-(CH_2)_{n''}-R^3-O-CH=CH_2 \; ;$$

$$-(O)_{n'}-(CH_2)_{n''}-O-CH=CH-R^4$$

dans lesquelles:

- n' représente 0 ou 1 et n" un entier compris entre 1 et 5
- $R^3$ représente:

  - un radical alkylène linéaire, ramifié ou cyclique en $C_1$-$C_{12}$, éventuellement substitué,

- ou un radical arylène en $C_5$-$C_{12}$, éventuellement substitué, et

• $R^4$ représente un radical alkyle linéaire ou ramifié en $C_1$-$C_6$.

**12.** Composition selon la revendication 10 ou 11, **caractérisée en ce qu'**au moins l'un des symboles $R^1$des polyorganosiloxane utilisés représente un radical phényle, tolyle ou dichorophènyle.

**13.** Composition selon l'une des revendications 7 à 12, **caractérisée en ce qu'**elle comprend en outre un photoamorceur cationique.

**14.** Résine susceptible d'être obtenue à partir d'une composition selon l'une quelconque des revendications 7 à 13.

**15.** Polymère susceptible d'être obtenu à partir d'une composition selon l'une quelconque des revendications 7 à 13.

**16.** Revêtement à base d'une résine selon la revendication 14.

**17.** Revêtement selon la revendication 16, **caractérisé en ce que** le revêtement est un vernis, un revêtement adhésif, un revêtement anti-adhérent et/ou une encre.

**18.** Composition dentaire obtenue à partir d'une composition selon l'une quelconque des revendications 7 à 13.

**19.** Composition dentaire selon la revendication 18, **caractérisée en ce qu'**il s'agit d'une prothèse dentaire ou un matériau de restauration dentaire.

**20.** Objet dont une surface au moins est revêtue d'une résine selon la revendication 14.

**21.** Procède de polymérisation et/ou réticulation d'une composition selon l'une quelconque des revendications 7 à 13 comprenant les étapes suivantes :

(1) on expose la composition à une source de chauffage,
(2) on polymérise et/ou réticule.

**22.** Utitisation selon l'une des revendications 1 à 6, **caractérisé en ce que** les monomères, oligomères et/ou de polymères de nature polyorganosiloxane à groupements organofonctionnels sont tels que définis en revendications 7 à 12.

## Patentansprüche

**1.** Verwendung als Beschleuniger für die thermisch aktivierbare Polymerisation und/oder Vernetzung von Polyorganosiloxanmonomeren, -oligomeren und/oder -polymeren mit organofunktionellen Gruppen mittels wenigstens eines Borderivates der Formel (I) :

$$(A)_xB(R')_y \qquad (I)$$

worin:

die Symbole R' gleich oder verschieden sind und darstellen:

- einen geraden oder verzweigten Alkyl- oder Alkenylrest mit $C_1$-$C_{12}$, vorzugsweise $C_1$-$C_8$, eventuell substituiert, mit wenigstens einem elektronenanziehenden Rest, insbesondere einem Halogenatom oder einer elektronenanziehehden Gruppe,
- einen linearen oder verzweigten Alkoxyrest mit $C_2$-$C_{12}$, vorzugsweise $C_1$-$C_8$, eventuell substituiert mit wenigstens einem elektronenanziehenden Rest, insbesondere einem Halogenatom oder einer elektronenanziehenden Gruppe,
- einen Phenylrest, substituiert mit wenigstens einem elektronenanziehenden Rest, insbesondere einem

Halogenatom oder einer elektrozienanziehenden Gruppe,

- einen Arylrest, der wenigstens zwei aromatische Ringe, wie Biphenyl, Naphthyl, enthält, eventuell substituiert mit wenigstens einem elektronenanziehenden Rest, insbesondere einem Halogenatom oder einer elektronenanziehenden Gruppe,
- einen Rest $-C_2H_4-Si(Q)_3$, wobei die Symbole Q, gleich oder verschieden, darstellen eine Alkyl- oder Alkoxygruppe mit $C_1$ bis $C_{10}$ oder ein Siloxanoligomeres mit weniger als 10 Siliziumatomen, gegebenenfalls substituiert mit einem Rest der Formel $B(R')_2$, wobei R' wie oben definiert ist, oder
- zwei Gruppen R' untereinander derart verbunden sein können, daß sie mit dem Boratom, an das sie gebunden sind, einen Ring mit 5 oder 10 Atomen, wobei dieser Ring gesättigt, ungesättigt, verbrückt oder aromatisch sein kann und ein oder mehrere Heteroatome, ausgewählt unter Sauerstoff-, Stickstoff- und Boratomen umfassen kann, wobei das in diesem Ring vorhandene Boratom selbst durch einen Rest, wie für A oder R' in der allgemeinen Formel I definiert, substituiert sein kann,

die Symbole A unabhängig voneinander darstellen:

- ein Wasserstoffatom,
- ein Halogenatom,
- einen Hydroxylrest,

x 0 oder die ganze Zahl 1 oder 2 darstellt, und
y eine ganze Zahl 1, 2 oder 3 darstellt, wobei die Summe von n+y = 3 ist,

sowie seiner solvatisierten Form oder Formen.

**2.** Verwendung nach Anspruch 1, **dadurch gekennzeichnet, daß** die Symbole R' derart ausgewählt sind, daß sie dem Boratom, an das sie gebunden sind, ein ausreichendes sterisches Umfeld erteilen, um ihm einen wirksamen Schutz gegen Oxidations- oder Hydratationserscheinungen sicherzustellen.

**3.** Verwendung nach Anspruch 1 oder 2, **dadurch gekennzeichnet, daß** die Symbole R' gemeinsam mit den Symbolen A zu einem $\sigma_p$ wenigstens gleich demjenigen von 3 Resten ($C_6H_4F$) beitragen.

**4.** Verwendung nach einem der Ansprüche 1 bis 3, **dadurch gekennzeichnet, daß** dieser Beschleuniger der allgemeinen Formel Ia) entspricht:

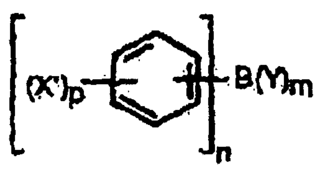

(Ia)

in der

n eine ganze Zahl zwischen 1 und 3 darstellt, und
m eine ganze Zahl zwischen 0 und 2 darstellt,

wobei die Summe von n und m = 3 ist,
die Symbole Y gleich oder verschieden sind und darstellen:

- ein Wasserstoffatom,
- eine Hydroxylgruppe,
- ein Halogenatom,
- einen geraden oder verzweigten Alkyl- oder Alkenylrest mit $C_1$-$C_{12}$. vorzugsweise $C_1$-$C_8$, bevorzugt substituiert mit wenigstens einem Halogenatomrest,
- einen linearen oder verzweigten Alkoxyrest mit $C_1$-$C_{12}$, vorzugsweise $C_1$-$C_8$, substituiert mit einem Halogenatomrest,
- einen Rest $-C_2H_4-Si(Q)_3$, wobei die Symbole Q eine Alkyloder Alkoxygruppe mit $C_1$ bis $C_{10}$ oder ein Siloxan-

oligomeres mit weniger als 10 Siliziumatomen, gegebenenfalls Substituiert mit einem Rest der Formel $B(R')_2$, wobei R' wie oben definiert ist, darstellen, oder

- zwei Gruppen Y untereinander derart verbunden sein können, daß sie mit dem Boratom, an das sie gebunden sind, einen Ring mit $C_5$-$C_{10}$ darstellen, wobei dieser Ring gesättigt, ungesättigt, verbrückt oder aromatisch sein kann und ein oder mehrere Heteroatome, ausgewählt unter Sauerstoff-, Stickstoff- und Boratomen umfassen kann, wobei das in diesem Ring vorhandene Boratom selbst durch einen Rest, wie für Y in der allgemeinen Formel (Ia) definiert, substituiert sein kann,

die Symbole X' gleich oder verschieden sind und darstellen:

- ein Halogenatom,
- einen linearen oder verzweigten, mono- oder polycyclischen, gesättigten oder ungesättigten oder aromatischen und bevorzugt durch wenigstens einen Halogenatomrest substituierten Kohlenwasserstoffrest mit $C_1$-$C_{12}$, vorzugsweise $C_1$-$C_8$, oder einen linearen- oder verzweigten, mono-, poly- oder perhalogenierten Alkylrest mit $C_1$-$C_{12}$, und
- die Indices p gleich oder verschieden sind und eine ganze Zahl zwischen 0 und 5 darstellen, wobei bevorzugt wenigstens eines der Symbole p oberhalb 3 und bevorzugt gleich 5 ist.

**5.** Verwendung nach einem der Ansprüche 1 bis 4, **dadurch gekennzeichnet., daß** dieser Beschleuniger aus den folgenden Verbindungen ausgewählt ist:

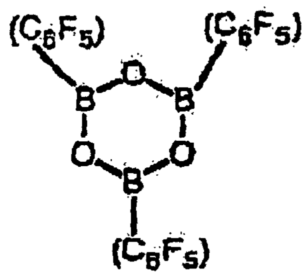
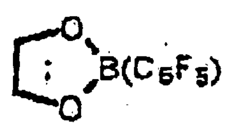
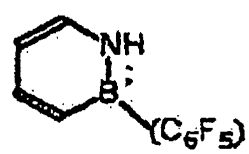

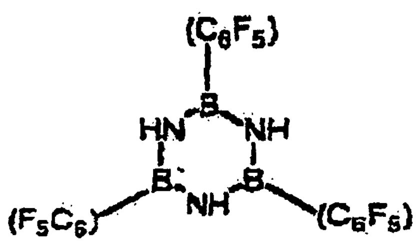
;

$(C_5F_4)(C_6F_5)_2B$ ; $(C_5F_4)_3B$ ; $(C_6F_5)BF_2$ ; $BF(C_6F_5)_2$ ; $B(C_6F_5)_3$ ; $BCl_2(C_6F_5)$ ; $BCl(C_6F_5)_2$ ; $B(C_6H_5)(C_6F_5)_2$ ;

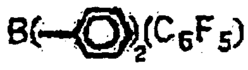
;

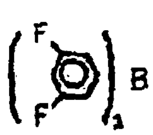
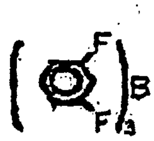
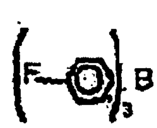
;

$[C_6H_4(mCF_3)]_3B$ ; $[C_6H_4(pOCF_3)]_3B$ ;

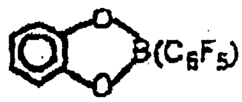 ;

$(C_6F_5)B(OH)_2$ ; $(C_6F_5)_2BOH$ ; $(C_6F_5)_2BH$ ; $(C_6F_5)BH_2$ ;

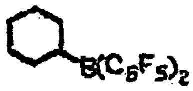 ;

$(C_7H_{11})B(C_6F_5)_2$ ;$(C_8H_{14}B)(C_6F_5)$ ;

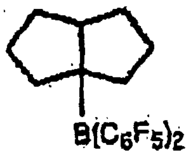 ; $(C_6F_5)_2B(OC_2H_5)$ ; 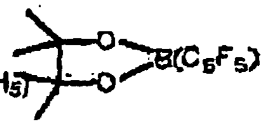 ;

$(C_6F_5)_2B\text{-}CH_2\ CH_2\ SI(CH_3)_3$ :

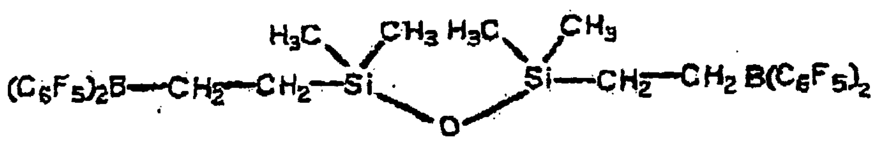

;

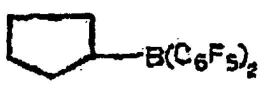 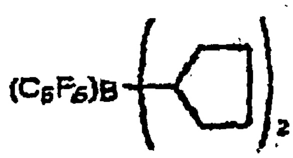 ;

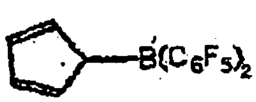 ; 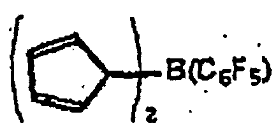 .

**6.** Verwendung nach einem der vorhergehenden Ansprüche, **dadurch gekennzeichnet, daß** dieser Beschleuniger in Lösung in einem Lösungsmittel verwendet wird.

**7.** Polymerisierbare und/oder vernetzbare Zusammensetzung,
**dadurch gekennzeichnet, daß** die Zusammensetzung wenigstens ein Polyorganosiloxan-Monomeres, -Oligomeres und/oder -Polymeres mit organofunktionellen Gruppen und eine wirksame Menge wenigstens eines thermisch aktivierbaren Beschleunigers entsprechend einem der vorhergehenden Ansprüche umfaßt.

**8.** Zusammensetzung nach Anspruch 7, **dadurch gekennzeichnet, daß** der Beschleuniger in einer Menge von

0,0001 bis 5 Gew.-Teilen auf 100 Gew.-Teile des Trockenmaterials aus Polyorganosiloxan-Monomeren, -Oligomeren und/oder -Polymeren mit organofunktionelleh Gruppen verwendet wird.

**9.** Zusammensetzung nach Anspruch 7 oder 8, **dadurch gekennzeichnet, daß** die Polyorganosiloxan-Monomeren, -Oligomereir und/oder -Polymeren mit organofunktionellen Gruppen als organofunktionelle Gruppen Epoxyd-, Oxetan-, Dioxolan- und/oder Alkenylethergruppen besitzen.

**10.** Zusammensetzung nach Anspruch 7, 8 oder 9, **dadurch gekennzeichnet, daß** die Polyorganosiloxan-Monomeren, -Oligomeren und/oder -Polymeren mit organofunktionellen Gruppen aus Einheiten der Formel (II) und terminiert mit Einheiten der Formel (III) oder cyclischen Einheiten de Formel (II), wie unten angegeben, gebildet sind:.

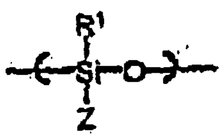 (II) 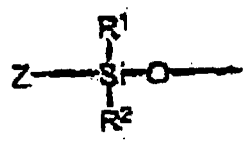 (III)

worin:

die Symbole $R^1$ und $R^2$ vergleichbar oder verschieden sind und darstellen:

- einen linearen oder verzweigten Alkylrest mit 1 bis 8 Köhlenstoffatomen, eventuell substituiert durch wenigstens ein Halogen,
- einen Cycloalkylrest mit zwischen 5 und 8 cyclischen Kohlenstofftomen, eventuell substituiert,
- einen Arylrest mit zwischen 6 und 12 Kohlenstoffatomen, der substituiert sein kann;
- einen Aralkylrest, der einen Alkylteil mit zwischen 5 und 14 Kohlenstoffatomen und einen Arylteil mit zwischen 6 und 12 Kohlenstoffatomen, eventuell substituiert auf dem Arylteil mit Halogenen, Alkylen und/oder Alkoxylen mit 1 bis 3 Kohlenstoffatomen,

die Symbole Z vergleichbar oder verschieden sind und darstellen:

- eine Gruppe $R^1$ und/oder $R^2$,
- einen Wasserstoffrest,
- und/oder eine vernetzbare organofunktionelle Gruppe, bevorzugt eine epoxyfunktionelle, oxetanfunktionelle, dioxolanfunktionelle und/oder alkenyletherfunktionelle Gruppe, gebunden an Silizium des Polyorganosiloxans unter Zwischenschaltung eines zweiwertigen Restes mit 2 bis 20 Kohlenstoffatomen, der wenigstens ein Heteroatom enthalten kann,

wobei wenigstens eines der Symbole Z eine organische, funktionelle, vernetzbare Gruppe darstellt.

**11.** Zusammensetzung nach Anspruch 10, **dadurch gekennzeichnet, daß** die organofunktionellen Gruppen ausgewählt sind aus:

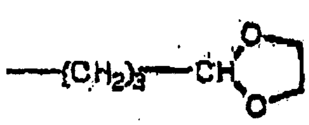 ; 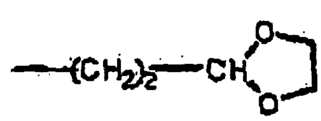

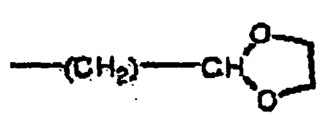

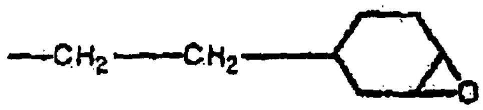 ; 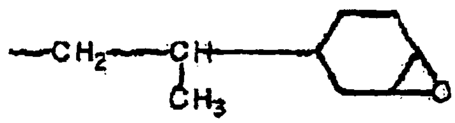

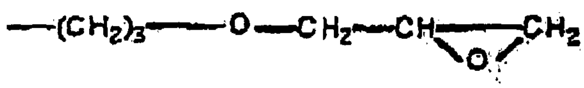 ; 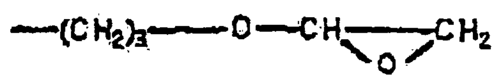

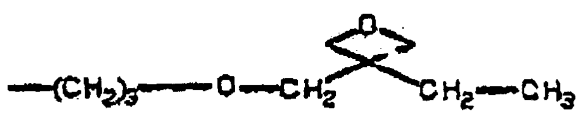

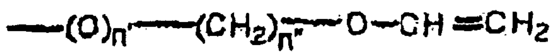 ;

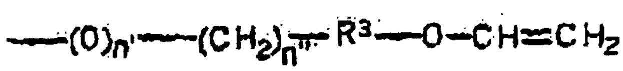 ;

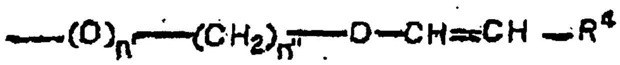

worin:

- n' 0 oder 1 darstellt und n" eine ganze Zahl zwischen 1 und 5 darstellt,
- $R^3$ darstellt:
  - einen linearen, verzweigten oder cyclischen Alkylenrest mit $C_1$-$C_{12}$, eventuell substituiert, oder
  - einen Arylenrest mit $C_5$-$C_{12}$, eventuell substituiert, und
- $R^4$ einen linearen oder verzweigten Alkylrest mit $C_1$-$C_6$ darstellt.

**12.** Zusammensetzung nach Anspruch 10 oder 11, **dadurch gekennzeichnet, daß** wenigstens eines der Symbole $R^1$ der dargestellten verwendeten Polyorganosiloxane einen Phenyl-, Tolyloder Dichlorphenylrest darstellt.

**13.** Zusammensetzung nach einem der Ansprüche 7 bis 12, **dadurch gekennzeichnet, daß** sie weiter einen kationischen Photobeschleuniger umfaßt.

**14.** Harz, erhältlich aus einer Zusammensetzung nach irgendeinem der Ansprüche 7 bis 13.

**15.** Polymeres, erhaltlich aus einer Zusammensetzung nach irgendeinem der Ansprüche 7 bis 13.

**16.** Überzug auf Basis eines Harzes nach Anspruch 14.

**17.** überzug nach Anspruch 16, **dadurch gekennzeichnet, daß** der Überzug ein Lack, ein Haftüberzug, ein Antihaftüberzug und/oder ein Anstrich ist.

**18.** Dentalzusammensetzung, erhalten aus einer Zusammensetzung nach irgendeinem der Ansprüche 7 bis 13.

**19.** Dentalzusammensetzurig nach Anspruch 18, **dadurch gekennzeichnet, daß** es sich um eine Zahnprothese oder um ein Zahnrestaurationsmaterial handelt.

**20.** Gegenstand, von dem wenigstens eine Oberfläche mit einem Harz nach Anspruch 14 bedeckt ist.

**21.** Verfahren zur Polymerisation und/oder Vernetzung einer Zusammensetzung nach irgendeinem der Ansprüche 7 bis 13, umfassend die folgenden Stufen:

(1) die Zusammensetzung wird einer Erwärmungsquelle ausgesetzt,
(2) es wird polymerisiert und/oder vernetzt.

**22.** Verwendung nach einem der Ansprüche 1 bis 6, **dadurch gekennzeichnet, daß** die Polyorganosiloxan-Monomeren, -Oligomeren und/oder -Polymeren mit organofunktionellen Gruppen, wie in den Ansprüche 7 bis.12 definiert, sind.

## Claims

**1.** Use as an initiator, for the heat-activatable polymerization and/or crosslinking of monomers, oligomers and/or polymers of polyorganosiloxane nature containing organofunctiorial groups, of at least one boron derivative of formula (I)

$$(A)_xB(R')_y \qquad (I)$$

in which

- the symbols R' are identical or different and represent
  - a linear or branched $C_1$-$C_{12}$ and preferably $C_1$-$C_8$ alkyl or alkenyl radical, optionally substituted with at least one electron-withdrawing element, especially a halogen atom, or an electron-withdrawing group,
  - a linear or branched $C_1$-$C_{12}$ and preferably $C_1$-$C_8$ alkoxy radical, optionally substituted with at least one electron-withdrawing element, especially a halogen atom, or an electron-withdrawing group,
  - a phenyl radical substituted with at least one electron-withdrawing element, especially a halogen atom; or an electron-withdrawing group,
  - an aryl radical containing at least two aromatic nuclei, such as biphenyl or naphthyl, optionally substituted with at least one electron-withdrawing element, especially a halogen atom, or an electron-withdrawing group,
  - a radical $-C_2H_4-Si(Q)_3$ with the symbols Q, which may be identical or different, representing a $C_1$ to $C_{10}$ alkyl or alkoxy group or a siloxane oligomer of less than 10 silicon atoms, where appropriate substituted with a radical of formula $B(R')_2$ with R' as defined above, or
  - two groups R' may be linked together so as to form, with the boron atom to which they are attached, a ring containing 5 or 10 atoms, the said ring possibly being saturated, unsaturated, bridged or aromatic and comprising one or more hetero atoms chosen from oxygen,nitrogen and boron atoms, the boron atom present in the said ring possibly being itself substituted with a radical as defined for A or R' in general formula I,
- the symbols A represent, independently of each other
  - a hydrogen atom,
  - a halogen atom, or
  - a hydroxyl radical,
  - x represents 0 or the integer 1 or 2 and y represents the integer 1, 2 or 3, the sum of n+y being equal to 3, and

the solvated form(s) thereof.

**2.** Use according to Claim 1, **characterized in that** the symbols R' are chosen so as to give the boron atom to which they are attached sufficient steric hindrance to afford it efficient protection against oxidation and/or hydration.

**3.** Use according to Claim 1 or 2, **characterized in that** the symbols R' contribute overall with the symbols A to a $\sigma_p$ at least equal to that of 3 ($C_6H_4F$) radicals.

**4.** Use according to one of Claims 1 to 3, **characterized in that** the said initiator corresponds to the general formula (Ia)

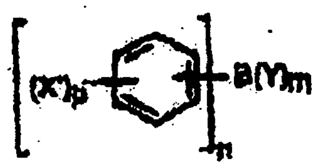

(Ia)

in which

- n represents an integer between 1 and 3 and m represents an integer between 0 and 2, the sum of n and m being equal to 3,
- the symbols Y are identical or different and represent
  - a hydrogen atom,
  - a hydroxyl group,
  - a halogen atom,
  - a linear or branched $C_1$-$G_{12}$ and preferably $C_1$-$C_8$ alkyl or alkenyl radical, preferably substituted with at least one halogen atom element,
  - a linear or branched $C_1$-$C_{12}$ and preferably $C_1$-$C_8$ alkoxy radical, preferably substituted with at least one halogen atom element,
  - — $C_2H_4$—$Si(Q)_3$ with Q representing a $C_1$ to $C_{10}$ alkyl or alkoxy group or a siloxane oligomer containing less than 10 silicon atoms, where appropriate substituted with a radical of formula $B(R')_2$ with R' as defined above, or
  - two groups Y may be linked together so as to form, with the boron atom to which they are attached, a $C_5$—$C_{10}$ ring, the said ring possibly being saturated, unsaturated, bridged or aromatic and comprising one or more hetero atoms chosen from oxygen, nitrogen and boron atoms, the boron atom present in the said ring possibly being itself substituted with a radical as defined for Y in general formula (Ia), and

- the symbols X' are identical or different and represent
  - a halogen atom,
  - a linear, branched, monocyclic or polycyclic, saturated, unsaturated or aromatic $C_1$-$G_{12}$ and preferably $C_1$-$C_8$ hydrocarbon-based radical, preferably substituted with at least one halogen atom element, or
  - a linear or branched, mono-, poly- or per-halogenated $C_1$-$C_{12}$ alkyl radical, and
  - the indices p are identical or different and represent an integer between 0 and 5, preferably with at least one of the symbols p being greater than 3 and more preferably equal to 5.

**5.** Use according to one of Claims 1 to 4, **characterized in that** the said initiator is chosen from the following compounds:

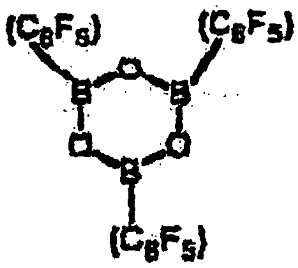 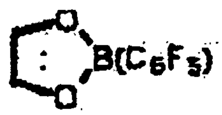 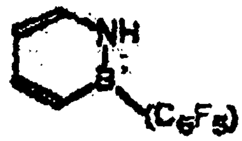

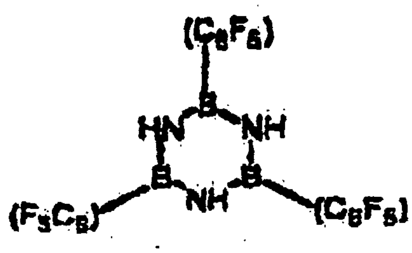 ;

$(C_5F_4)(C_6F_5)_2B$ ; $(C_5F_4)_3B$ ; $(C_6F_5)BF_2$ ; $BF(C_6F_5)_2$ ; $B(C_6F_5)_3$ ; $BCl_2(C_6F_5)$ ; $BCl(C_6F_5)_2$ ; $B(C_6H_5)(C_6F_5)_2$ ;

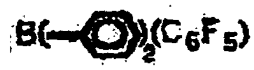

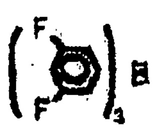 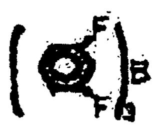 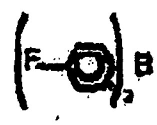 ;

$[C_6H_4(mCF_3)]_3B$ ; $[C_6H_4(pOCF_3)]_3B$ ;

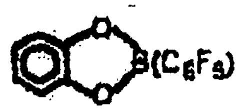 ;

$(C_6F_5)B(OH)_2$ : $(C_6F_5)_2BOH$ ; $(C_6F_5)_2BH$ ; $(C_6F_5)BH_2$

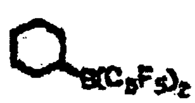 ;

$(C_7H_{11})B(C_6F_5)_2$ ; $(C_8H_{14}B)(C_6F_5)$ ;

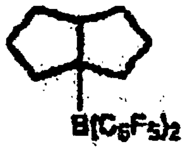

;

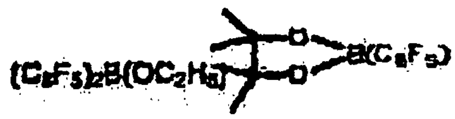

$(C_6F_5)_2B\text{-}CH_2\ CH_2\ Si(CH_3)_3$ ;

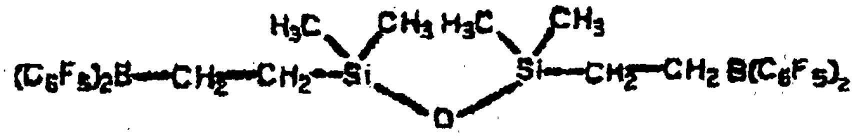

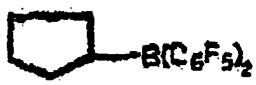

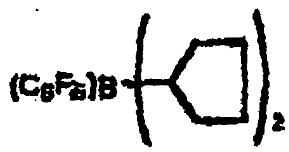

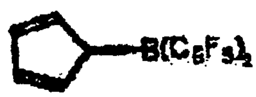

;

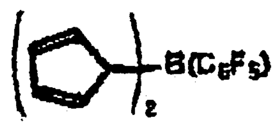

6. Use according to one of the preceding claims, **characterized in that** the said initiator is used in solution in a solvent.

7. Polymerizable and/or crosslinkable composition, **characterized in that** the composition comprises at least one polyorganosiloxane monomer, oligomer and/or polymer containing organofunctional groups and an effective amount of at least one heat-activatable initiator according to any one of the preceding claims.

8. Composition according to Claim 7, **characterized in that** the initiator is used in a proportion of from 0.0001 to 5 parts by weight per 100 parts by weight of solids as polyorganosiloxane monomers. oligomers and/or polymers containing organofunctional groups.

9. Composition according to Claim 7 or 8, **characterized in that** the monomers, oligomers and/or polymers of polyorganosiloxane nature contain epoxide, oxetane, dioxolane and/or alkenyl ethers as organofunctional groups.

10. Composition according to Claim 7, 8 or 9, **characterized in that** the polyorganosiloxane monomers, oligomers and/or polymers containing organofunctional groups consist of units of formula (II) ending with units of formula (III), or are cyclic and consist of units of formula (II) represented below:

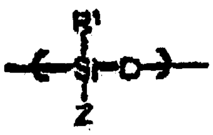 (II)

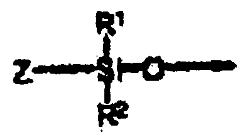 (III)

in which:

- the symbols $R^1$ and $R^2$ are identical or different and represent:

- a linear or branched alkyl radical containing 1 to 8 carbon atoms, optionally substituted with at least one halogen,
- an optionally substituted cycloalkyl radical containing between 5 and 8 ring carbon atoms,
- an aryl radical containing between 6 and 12 carbon atoms, which may be substituted;
- an aralkyl portion having an alkyl portion containing between 5 and 14 carbon atoms and an aryl portion containing between 6 and 12 carbon atoms, optionally substituted on the aryl portion with halogens, alkyls and/or alkoxys containing 1 to 3 carbon atoms,

- the symbols Z are identical or different and represent:

  - a group $R^1$ and/or $R^2$,
  - a hydrogen radical, and/or
  - a crosslinkable organofunctional group, preferably an epoxy-functional, oxetane-functional, dioxolane-functional and/or alkenyl ether-functional group, linked to the silicon of the polyorganosiloxane via a divalent radical containing from 2 to 20 carbon atoms and possibly containing at least one hetero atom,
  - with at least one of the symbols Z representing a crosslinkable organofunctional group.

**11.** Composition according to Claim 10, **characterized in that** the organofunctional groups, are chosen from:

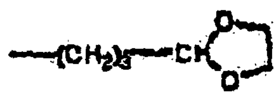 ; 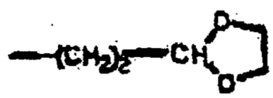

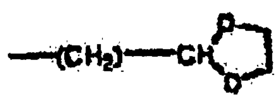

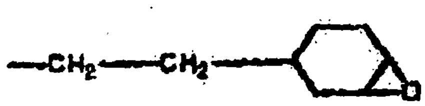 ; 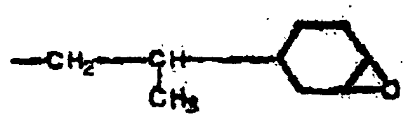

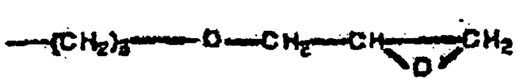 ; 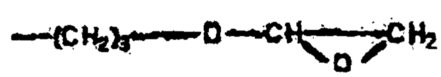

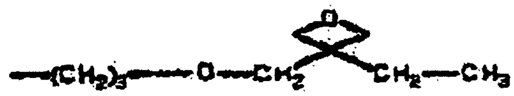

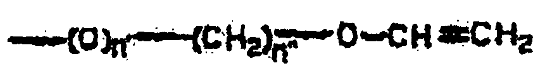 ;

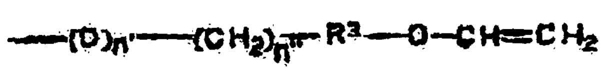 ;

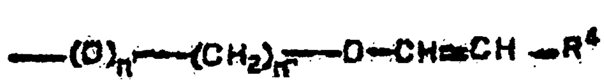

in which:

- n' represents 0 or 1 and n" represents an integer between 1 and 5,
- $R^3$ represents :
  - an optionally substituted linear, branched or cyclic $C_1$-$C_{12}$ alkylene radical,or
  - an optionally substituted $C_5$-$C_{12}$ arylene radical, and
- $R^4$ represents a linear or branched $C_1$-$C_8$ alkyl radical.

**12.** Composition according to Claim 10 or 11, **characterized in that** at least one of the symbols $R^1$ of the polyorganosiloxanes used represents a phenyl, tolyl or dichlorophenyl radical.

**13.** Composition according to one of Claims 7 to 12, **characterized in that** it also comprises a cationic photoinitiator.

**14.** Resin that may be obtained from a composition according to any one of Claims 7 to 13.

**15.** Polymer that may be obtained from a composition according to any one of Claims 7 to 13.

**16.** Coating based on a resin according to Claim 14.

**17.** Coating according to Claim 16, **characterized in that** the coating is a varnish, an adhesive coating, a release coating and/or an ink.

**18.** Dental composition obtained from a composition according to any one of Claims 7 to 13.

**19.** Dental composition according to Claim 18, **characterized in that** it is a dental prosthesis or a dental restoration material.

**20.** Article, at least one surface of which is coated with a resin according to Claim 14.

**21.** Process for polymerizing and/or crosslinking a composition according to any one of Claims 7 to 13, comprising the following steps:

(1) the composition is exposed to a heating source,
(2) polymerization and/or crosslinking is performed.

**22.** Use according to one of Claims 1 to 6, **characterized in that** the monomers, oligomers and/or polymers of polyorganosiloxane nature containing organofunctional groups are as defined in Claims 7 to 12.